# EUROPEAN PATENT SPECIFICATION

(11) **EP 3 362 056 B1**
(45) Date of publication and mention of the grant of the patent: **24.02.2021**
(21) Application number: 16784831.6
(22) Date of filing: 14.10.2016
(51) Int. Cl.: A61K 31/131, A61K 31/136, A61K 31/137, A61K 31/4453, A61K 31/5375, A61P 35/00, G01N 33/50

(54) **AMINOTHIOLESTER COMPOUNDS OR PHARMACEUTICALLY ACCEPTABLE SALTS THEREOF, FOR USE IN THE TREATMENT OF CANCER**
AMINOTHIOLESTERVERBINDUNGEN ODER PHARMAZEUTISCH UNBEDENKLICHE SALZE DAVON ZUR VERWENDUNG BEI DER BEHANDLUNG VON KREBS
COMPOSÉS AMINOTHIOLESTERS OU LEURS SELS PHARMACEUTIQUEMENT ACCEPTABLES, DESTINÉS À ÊTRE UTILISÉS POUR LE TRAITEMENT DU CANCER

(30) Priority: 15.10.2015 EP 15306649
(43) Date of publication of application: 22.08.2018
(73) Proprietor: Advanced Biodesign, 69800 Saint Priest (FR)
(72) Inventor: CEYLAN, Ismail, 69800 Saint-Priest (FR); QUASH, Gerry, 69800 Saint-Priest (FR); PEREZ-ALEA, Mileidys, 69800 Saint-Priest (FR); MARTIN, Guillaume, 69800 Saint-Priest (FR)
(74) Representative: Lavoix
(86) International application number: PCT/EP2016/074697
(87) International publication number: WO 2017/064247

(56) References cited:
- US-A1- 2007 032 476
- HELENE PELICANO ET AL: "ROS stress in cancer cells and therapeutic implications", DRUG RESISTANCE UPDATES, vol. 7, no. 2, April 2004 (2004-04), pages 97-110, XP055060447, ISSN: 1368-7646, DOI: 10.1016/j.drup.2004.01.004
- QUASH G ET AL: "Aldehyde dehydrogenase inhibitors: alpha,beta-Acetylenic N-substituted aminothiolesters are reversible growth inhibitors of normal epithelial but irreversible apoptogens for cancer epithelial cells from human prostate in culture", EUROPEAN JOURNAL OF MEDICINAL CHEMISTRY, EDITIONS SCIENTIFIQUE ELSEVIER, PARIS, FR, vol. 43, no. 5, May 2008 (2008-05), pages 906-916, XP022639683, ISSN: 0223-5234, DOI: 10.1016/J.EJMECH.2007.06.004 [retrieved on 2008-05-01] cited in the application
- DUNYAPORN TRACHOOTHAM ET AL: "Selective killing of oncogenically transformed cells through a ROS-mediated mechanism by [beta]-phenylethyl isothiocyanate", CANCER CELL, vol. 10, no. 3, September 2006 (2006-09), pages 241-252, XP055244751, US ISSN: 1535-6108, DOI: 10.1016/j.ccr.2006.08.009
- B. KUMAR ET AL: "Oxidative Stress Is Inherent in Prostate Cancer Cells and Is Required for Aggressive Phenotype", CANCER RESEARCH, vol. 68, no. 6, 15 March 2008 (2008-03-15) , pages 1777-1785, XP055244614, US ISSN: 0008-5472, DOI: 10.1158/0008-5472.CAN-07-5259
- GIOVANNI L. BERETTA ET AL: "Unravelling "off-target" effects of redox-active polymers and polymer multilayered capsules in prostate cancer cells", NANOSCALE, vol. 7, no. 14, 17 March 2015 (2015-03-17) , pages 6261-6270, XP055332472, United Kingdom ISSN: 2040-3364, DOI: 10.1039/C4NR07240E

## Description

The present invention relates to the treatment of cancer in a subject, wherein cancer cells of said subject overproduce H₂O₂ and have a level of GSH below 0,5 nmol for 25 000 cells; with aminothiolester compounds or pharmaceutically acceptable salts thereof, in particular with S-methyl 4-(dimethylamino)-4-methylpent-2-ynethioate or a pharmaceutically acceptable salt thereof, more particularly with 4-(Dimethylamino)-4-methyl-2-pentynethioic acid S-methyl ester fumarate. It also relates to a method for selecting a subject suffering from a cancer and who will most likely benefit from a treatment with aminothiolester compounds or pharmaceutically acceptable salts thereof, in particular with S-methyl 4-(dimethylamino)-4-methylpent-2-ynethioate or a pharmaceutically acceptable salt thereof, more particularly with 4-(Dimethylamino)-4-methyl-2-pentynethioic acid S-methyl ester fumarate.

### Background

The redox balance of cells is key to normal cell physiology. It is maintained by 3 systems: GSH/GSSG, NADPH/NADP; Thioredoxin (red) /Thioredoxin (oxd). Of these 3 systems, GSH/GSSG is the most widely studied for its implication in diseased states and for the development of rational therapeutic approaches (Townsend, A.J., Leone-Kabler, S., Haynes, R.L., Wu, Y., Szweda, L., and Bunting, K.D. (2001). Selective protection by stably transfected human ALDH3A1 (but not human ALDH1A1) against toxicity of aliphatic aldehydes in V79 cells. 130-132, 261-273). The diseased states associated with an imbalance in GSH/GSSG include major pathologies like cancers (Estrela, J.M., Ortega, A., and Obrador, E. (2006). Glutathione in cancer biology and therapy. Crit. Rev. Clin. Lab. Sci. 43, 143-181; O'Brien, M.L., and Tew, K.D. (1996). Glutathione and related enzymes in multidrug resistance. Eur. J. Cancer Oxf. Engl. 1990 32A, 967-978). Their one common aetiology is oxidative stress brought about by ROS and/or Reactive Nitrogen Species (RNS) that first cause a decrease in Glutathione (GSH) due to the direct detoxification of ROS and RNS. This initial decrease in GSH is followed subsequently by a compensatory increase in GSH synthesis that cells bring into play in order to continue the detoxification of ROS/RNS and of newly formed electrophilic products such as 4-hydroxynonenal (HNE) and malondialdehyde (MDA)) produced by ROS attack on cellular lipids (Esterbauer, H., Schaur, R.J., and Zollner, H. (1991). Chemistry and biochemistry of 4-hydroxynonenal, malonaldehyde and related aldehydes. 11, 81-128).

Increases in ROS have been reported in different human cancers (Reuter, S., Gupta, S.C., Chaturvedi, M.M., and Aggarwal, B.B. (2010). Oxidative stress, inflammation, and cancer: how are they linked? Free Radic. Biol. Med. 49, 1603-1616).

The GSH paradox in cancer cells is that instead of the deficit in intracellular GSH that would have been expected, it is precisely the opposite that was found experimentally in many different cancer cells (Estrela, J.M., Ortega, A., and Obrador, E. (2006). Glutathione in cancer biology and therapy. Crit. Rev. Clin. Lab. Sci. 43, 143-181). But this increase in GSH has negative therapeutic repercussions as it protects cancer cells from chemo and radio therapies (Carretero, J., Obrador, E., Esteve, J.M., Ortega, A., Pellicer, J.A., Sempere, F.V., and Estrela, J.M. (2001). Tumoricidal activity of endothelial cells. Inhibition of endothelial nitric oxide production abrogates tumor cytotoxicity induced by hepatic sinusoidal endothelium in response to B16 melanoma adhesion in vitro. J. Biol. Chem. 276, 25775-25782).

In addition, if low levels of GSH must be obtained in cancer cells for chemotherapy to be effective, this is not the case for normal cells for not inducing collateral damage thereto.

The therapeutic approaches that are presently being used to lower cellular GSH in order to combat the chemoresistance of cancer cells, target GSH itself or the enzymes involved in GSH synthesis, GSH degradation and GSH efflux. There are already 10 GSH-lowering compounds that are in phases I, II and III of clinical trials as anticancer agents (Tew,K. and Townsend D (2011) Redox platforms in cancer drug discovery and development. Curr.Opin. Chem.Biol. 15, 156-161).

However they all have to be administered in combination with standard anti-cancer drugs, e.g. cyclophosphamide, taxol, vincristine, melphalan, etc.

Furthermore, the enzymes targeted by these GSH-lowering drugs are those involved in GSH synthesis (gamma glutamyl cysteine ligase), GSH degradation (gamma - glutamyl transpeptidase) and GSH efflux (GSH-S- transferase). These same enzymes are however essential for protecting normal cells from ROS attack. Hence, there is a strong possibility of collateral damage to normal cells as the drugs cannot be delivered selectively to cancer cells and to cancer cells only.

In view of this, there is a need to find other therapeutic solutions which specifically and selectively target cancer cells.

### Description of the invention

The inventors of the present invention have unexpectedly found that aminothiolester compounds or pharmaceutically acceptable salts thereof, in particular the S-methyl 4-(dimethylamino)-4-methylpent-2-ynethioate or a pharmaceutically acceptable salt thereof, are able to treat cancer in a subject, wherein cancer cells of said subject overproduce H₂O₂. In particular, they found that aminothiolester compounds or pharmaceutically acceptable salts thereof, in particular the S-methyl 4-(dimethylamino)-4-methylpent-2-ynethioate or a pharmaceutically acceptable salt thereof, are able to treat cancer in a subject, wherein cancer cells of said subject have an increased level of H₂O₂ of at least 200% compared with the basal level of H₂O₂ in normal cells originating from the same tissue as the cancer cells, and have a level of GSH below 0.5 nmol per 25 000 cells.

Without being bound by any theory, these compounds and in particular the S-methyl 4-(dimethylamino)-4-methylpent-2-ynethioate or a pharmaceutically acceptable salt thereof, would increase the levels of intra cellular metabolites that are produced by H₂O₂ attack, and, at the same time, GSH would thus be consumed in the detoxification of these electrophilic metabolites. As a result, insufficient GSH would be available to act as a scavenger of H₂O₂. Hence, levels of H₂O₂ should increase and should trigger-off the H₂O₂-dependent mechanisms in the mitochondrial (intrinsic) pathway of apoptosis. Thus, in addition to the high levels of H₂O₂ present in cancer cells due to dysfunction of the mitochondrial ETC (Electrons Transport Chain), these compounds and in particular the S-methyl 4-(dimethylamino)-4-methylpent-2-ynethioate or a pharmaceutically acceptable salt thereof, would induce additional expression of H₂O₂, leading cancer cells over their apoptogenic threshold. On the contrary, in normal cells that have not undergone attack by H₂O₂, intracellular GSH levels are already high (due to the absence of H₂O₂) so that there are no H₂O₂-induced electrophiles that will be targeted by the therapy used to increase them specifically in their cancer counterparts.

This new therapy presents the advantage that normal cells are spared because they have less suffered of H₂O₂ attack and that compared to the pertubators of GSH homoeostasis already known, such as piperlongumine (PLM) and partenolide (PTL) (Pei,S et al J.Biol.Chem. (2013) Targeting aberrant glutathione metabolism to eradicate human acute myelogenous leukemic cells; 288, 33542-33548) which are in clinical trials and are used in combination with standard chemotherapeutic agents, the compounds according to the invention and in particular the S-methyl 4-(dimethylamino)-4-methylpent-2-ynethioate or a pharmaceutically acceptable salt thereof, are effective as a single-drug regimen.

The present invention thus relates to a compound of formula (I): 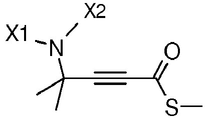 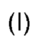 wherein X1 and X2, identical or different, are chosen among a C₁-C₇ alkyl group, a phenyl, a benzyl, or X1 and X2 together with the nitrogen atom to which they are linked form an heterocycle, in particular a piperidine or a morpholine; or a pharmaceutical acceptable salt thereof;
for use for the treatment of cancer in a subject, wherein cancer cells of said subject:
- have an increased level of H₂O₂ of at least 200% compared with the basal level of H₂O₂ in normal cells originating from the same tissue as the cancer cells, and
- have a level of GSH below 0.5 nmol per 25 000 cells.

In particular, cancer cells of said subject have also a MDA-adducts level above 75 ng per µg of total protein and/or a HNE-adducts level above 1 µg per µg of total protein, after *in vitro* treatment with a compound of formula (I) or a pharmaceutical acceptable salt thereof

In particular, said subject is identified by measuring the H₂O₂ level and the GSH level in cancer cells of said subject.

The present invention further relates to a method for selecting a subject suffering from a cancer and who will most likely benefit from a treatment with a compound of formula (I): 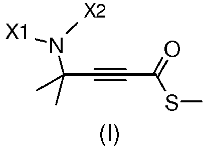 wherein X1 and X2, identical or different, are chosen among a C₁-C₇ alkyl group, a phenyl, a benzyl or X1 and X2 together with the nitrogen atom to which they are linked form an heterocycle, in particular a piperidine or a morpholine; or a pharmaceutically acceptable salt thereof;
wherein said method comprises:
a. measuring the H₂O₂ level in a cancer cells sample of said subject;
b. comparing the resulting level of step a. with a control value; and/or
c. measuring the GSH level in a cancer cells sample of said subject;
wherein:
- an increased level of H₂O₂ of at least 200% compared with the basal level of H₂O₂ in normal cells originating from the same tissue as the cancer cells, and
- a GSH level of said cancer cells sample of said subject below 0.5 nmol per 25 000 cells,
indicates that the subject is likely to benefit from a treatment with said compound or a pharmaceutically acceptable salt thereof.

In one embodiment, said method comprises:
a) measuring the H₂O₂ level in a cancer cells sample of said subject;
b) comparing the resulting level of step a. with a control value; and
c) measuring the GSH level in a cancer cells sample of said subject; and/or
d) measuring the MDA-adducts and/or HNE-adducts level after an *in vitro* treatment with a compound of formula (I) according to the invention or a pharmaceutical acceptable salt thereof in a cancer cells sample of said subject;
wherein:
- an increased level of H2O2 of at least 200% compared with the basal level of H₂O₂ in normal cells originating from the same tissue as the cancer cells, and
- a GSH level of said cancer cells sample of said subject below 0.5 nmol per 25 000 cells, and/or
- a MDA-adducts level above 75 ng per µg of total protein and/or a HNE-adducts level above 1 µg per µg of protein ,
indicates that the subject is likely to benefit from a treatment with said compound or a pharmaceutically acceptable salt thereof.

As described herein, "H₂O₂" means "hydrogen peroxide" and is a well-known by the man skilled in the art. It represents chemically reactive molecules containing oxygen. It is formed as a natural byproduct of the normal metabolism of oxygen and has important roles in cell signaling and homeostasis. However, during times of environmental stress (e.g., UV or heat exposure), it levels can increase dramatically. This may result in significant damage to cell structures. Cumulatively, this is known as oxidative stress. Hydrogen peroxide are also generated by exogenous sources such as ionizing radiation.

In particular, a H₂O₂ level higher than a value comprised between 2 000 and 400 000 Relative Fluorescence Intensity, for example between 10 000 and 100 000 Relative Fluorescence Intensity or 15 000 and 100 000 Relative Fluorescence Intensity, and more particularly a H₂O₂ level higher than 20 000 Relative Fluorescence Intensity, even more particularly higher than 21 598 Relative Fluorescence Intensity, indicates that the subject is likely to benefit from a treatment with a compound of formula (I) or a pharmaceutically acceptable salt thereof.

In particular, the level higher than a value comprised between 2 000 and 400 000 Relative Fluorescence Intensity, for example between 10 000 and 100 000 Relative Fluorescence Intensity or 15 000 and 100 000 Relative Fluorescence Intensity, and more particularly a H₂O₂ level higher than 20 000 Relative Fluorescence Intensity, even more particularly higher than 21 598 Relative Fluorescence Intensity, is measured with the Total ROS/superoxide detection kit (Enzo life science), more particularly as measured on an Appliskan fluorescence microplate reader (Thermo Scientific) (Ex/ Em= 488/520 nm and Ex/ Em= 550/610 nm).

If the cut-off level of H₂O₂ is given here and elsewhere in the description by measuring the fluorescence intensity, this method is nonexclusive and the cut-off level of H₂O₂ can be determined by any other method available to the man skilled in the art, the parameter determining where to put the cut-off being the correlation between the IC₅₀ of the product according to the invention and the level of H₂O₂. The IC₅₀ is a measure well-known to the man skilled in the art.

As described herein, "GSH" means "Glutathione "and is well known by the man skilled in the art. It is a tripeptide with a gamma peptide linkage between the carboxyl group of the glutamate side chain and the amine group of cysteine, and the carboxyl group of cysteine is attached by normal peptide linkage to a glycine.

In particular, in the scope of the present invention, the GSH level is below 0,5 nmol for 25 000 cells, in particular below 0,45 nmol for 25 000 cells and more particularly below 0,4 nmol for 25 000 cells.

The level of GSH is determined by any method available to the man skilled in the art. For example, the level of GSH is determined by luminescence, for example with the Promega GSH-Glo kit (Promega).

If the cut-off level of GSH is given here and elsewhere in the description by luminescence, this method is nonexclusive and the cut-off level of GSH can be determined by any other method available to the man skilled in the art

As described herein, "MDA" is used for "Malondialdehyde", an organic compound with the formula CH₂(CHO)₂. This reactive species is well known by the man skilled in the art and occurs naturally and is a marker lipids peroxidation in cells. In addition, a "MDA-adduct" according to the invention is an adduct formed between MDA and the proteins of the cancer cells as well as with the DNA of the cancer cells.

In particular, in the scope of the present invention, the MDA-adducts level after an *in vitro* treatment with a compound of formula (I) according to the invention or a pharmaceutical acceptable salt thereof is above 75ng per µg of total protein, in particular above 90 ng per µg of total protein and more particularly above 100 ng per µg of total protein. The level of MDA-adducts is determined by any method available to the man skilled in the art. For example, the level of MDA-adducts is determined by immuno-monitoring, for example with OxiSelect™ MDA-adduct competitive ELISA kit (CELL BIOLABS).

As described herein, "HNE" is used for "4-hydroxy-2-nonenal", an organic compound of formula C₉H₁₆O₂. This reactive species is well known by the man skilled in the art and occurs naturally and is a marker lipids peroxidation in cells.

In addition, a "HNE-adduct" according to the invention is an adduct formed between HNE and the proteins of the cancer cells as well as an adduct formed with the GSH of the cancer cells.

In particular, in the scope of the present invention, the HNE-adducts level after an *in vitro* treatment with a compound of formula (I) according to the invention or a pharmaceutical acceptable salt thereof is above 750 ng per µg of total protein, in particular above 900 ng per µgr of total protein and more particularly above 1µg per µg of total protein.

The level of HNE-adducts is determined by any method available to the man skilled in the art. For example, the level of HNE-adducts is determined by immuno-monitoring, for example with OxiSelect™ HNE-adduct competitive ELISA kit (CELL BIOLABS).

By "total protein" is meant the content of total protein of the cancer cell.

By *"in vitro* treatment with a compound of formula (I) according to the invention or a pharmaceutical acceptable salt thereof" is meant that the level of MDA-adducts and/or of HNE-adducts is measured after a step of treatment, *in vitro,* on a cancer cells sample of the subject, for example at the conditions described in the experimental section, taking into account that the doses of a compound of formula (I) according to the invention or of a pharmaceutical acceptable salt thereof which can be administered are until 60µM.

By a "C₁-C₇ alkyl group" is meant an aliphatic-hydrocarbon group which may be straight or branched having 1 to 7 carbon atoms in the chain unless specified otherwise. In particular, alkyl groups have 1 to 3 carbon atoms in the chain (C₁-C₃ alkyl). Branched means that one or more alkyl groups such as methyl, ethyl or propyl are attached to a linear alkyl chain. Exemplary alkyl groups include methyl, ethyl, n-propyl, i-propyl, n-butyl, t-butyl, 2,2-dimethylbutyl, n-pentyl, n-hexyl, octyl, in particular methyl.

In particular, the compound of formula (I) is a compound as mentioned above wherein X1 and X2, identical or different, are chosen among a methyl, a phenyl, a benzyl, at least one of X1 or X2 being a methyl, or wherein X1 and X2 together with the nitrogen atom to which they are linked form a piperidine or a morpholine.

More particularly, said compound is chosen from:
- S-methyl 4-methyl-4-(piperidin-1-yl)pent-2-ynethioate;
- S-methyl 4-[benzyl(methyl)amino]-4-methylpent-2-ynethioate;
- S-methyl 4-methyl-4-[methyl(phenyl)amino]pent-2-ynethioate;
- S-methyl 4-methyl-4-(morpholin-4-yl)pent-2-ynethioate; and
- S-methyl 4-(dimethylamino)-4-methylpent-2-ynethioate.

In a preferred embodiment, said compound is the S-methyl 4-(dimethylamino)-4-methylpent-2-ynethioate.

These compounds can be prepared according to methods well known by the man skilled in the art. In particular, these compounds can be prepared from the corresponding acetylenic amine treated successively by BuLi, COS and Mel. A detailed process of preparation can be found for example in G.Quash et al., European Journal of Medicinal Chemistry 43 (2008) 906-916, from which the content is incorporated by reference, in particular in the part 2 of the Material and Methods section.

S-methyl 4-(dimethylamino)-4-methylpent-2-ynethioate (CAS number 350229-29-7, formula weight: 185.29 g.mol⁻¹, formula: C₉H₁₅NOS), also known under DIMATE is a compound of formula (II):

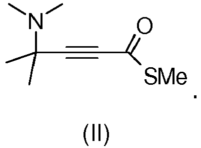

This compound and its process of preparation are for example described in the patent EP1296946 (in particular in example 1), from which the content is incorporated by reference.

By a "pharmaceutically acceptable salt" of a compound of formula (I), it is meant that the compound is modified by making acid or base salts thereof. The pharmaceutically acceptable salts include the conventional non-toxic salts or the quaternary ammonium salts of the compound, for example, from non-toxic inorganic or organic acids. For example, such conventional non-toxic salts include those such as fumarate, phosphate, citrate, chlorydrate, and the like. The pharmaceutically acceptable salts of of a compound of formula (I) can be synthesized from the parent compound by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418.

In particular, the compound of formula (I) is the S-methyl 4-(dimethylamino)-4-methylpent-2-ynethioate and its pharmaceutically acceptable salt is its fumarate salt i.e. the 4-(Dimethylamino)-4-methyl-2-pentynethioic acid S-methyl ester fumarate (formula weight: 301.4 g.mol⁻¹, formula: C₁₃H_{1g}NO₅S). Such fumarate salt can be prepared from S-methyl 4-(dimethylamino)-4-methylpent-2-ynethioate in anhydrous ether with addition of a solution of fumaric acid in anhydrous ethanol. The mono-fumarate salt is collected by filtration, washed with ether and dried.

In the scope of the invention, a compound of formula (I) or a pharmaceutically acceptable salt thereof, S-methyl 4-(dimethylamino)-4-methylpent-2-ynethioate or a pharmaceutical acceptable salt thereof, in particular 4-(Dimethylamino)-4-methyl-2-pentynethioic acid S-methyl ester fumarate, will be used interchangeably with the term "compound according to the invention".

The terms "treat", "treating", "treated" or "treatment", as used herein, refer to therapeutic treatment wherein the object is to eliminate or lessen symptoms. Beneficial or desired clinical results include, but are not limited to, elimination of symptoms, alleviation of symptoms, diminishment of extent of condition, stabilized (i.e., not worsening) state of condition, delay or slowing of progression of the condition, to the prevention of the onset, recurrence or spread of a disease or disorder, or of one or more symptoms thereof. In certain embodiments, the terms refer to the treatment with or administration of a compound provided herein prior to the onset of symptoms. The terms encompass the inhibition or reduction of a symptom of the particular disease. Subjects with familial history of a disease in particular are candidates for treatment regimens in certain embodiments. Also, subjects in whom a genetic disposition for the particular disease has been shown are candidates for treatment regimens in certain embodiments. In addition, subjects who have a history of recurring symptoms are also potential candidates for the treatment. In this regard, the term "treatment" may be interchangeably used with the term "prophylactic treatment."

As used herein and unless otherwise defined, "cancer" refers to the growth, division or proliferation of abnormal cells in the body. Cancers according to the invention are cancers in which an overproduction of H₂O₂ is observed in comparison to a control value, in particular cancers as defined in the claims in which there are both an overproduction of H₂O₂ observed in comparison to a control value and a level of GSH below 0,5 nmol for 25 000 cells. Such cancers include, but are not limited to bladder cancer, brain tumors, breast cancer, melanoma, multiple myeloma, leukemia, lymphoma, prostate cancer, cervical cancer, stomach cancer, liver cancer, tongue cancer, ovarian cancer, pancreatic cancer, renal cancer, pleuramesothelomia, osteosarcoma, muscle cancer, lung cancer, kidney cancer, head and neck cancer, colon cancer, blood cancer, cancers of the nervous central system and sarcoma; more particularly is chosen from bladder cancer, brain tumors, multiple myeloma, cervical cancer, stomach cancer, liver cancer, tongue cancer, ovarian cancer, pancreatic cancer, renal cancer, pleuramesothelomia, osteosarcoma, muscle cancer, kidney cancer, head and neck cancer, colon cancer, blood cancer, leukemia, cancers of the nervous central system and sarcoma.

As such, the present invention also relates to a compound of formula (I) or a pharmaceutically acceptable salt thereof, in particular S-methyl 4-(dimethylamino)-4-methylpent-2-ynethioate, or a pharmaceutically acceptable salt thereof, for use according to the invention or to a method according to the invention, wherein the cancer to treat is chosen from bladder cancer, brain tumors, breast cancer, melanoma, multiple myeloma, leukemia, lymphoma, prostate cancer, cervical cancer, stomach cancer, liver cancer, tongue cancer, ovarian cancer, pancreatic cancer, renal cancer, pleuramesothelomia, osteosarcoma, muscle cancer, lung cancer, kidney cancer, head and neck cancer, colon cancer, blood cancer, cancers of the nervous central system and sarcoma; in particular from bladder cancer, brain tumors, multiple myeloma, cervical cancer, stomach cancer, liver cancer, tongue cancer, ovarian cancer, pancreatic cancer, renal cancer, pleuramesothelomia, osteosarcoma, muscle cancer, kidney cancer, head and neck cancer, colon cancer, blood cancer, leukemia, cancers of the nervous central system and sarcoma.

Still particularly, said cancer is a chemoresistant and/or radioresistant cancer.

By "chemoresistant" is meant, a cancer as described herein against which chemotherapy doesn't work or stops working.

By "radioresistant" is meant, a cancer as described herein against which radiotherapy doesn't work or stops working.

In particular, said cancer is a cancer in which a MDA-adducts level above 75 ng per µg of total protein and/or a HNE-adducts level above 1 µg per µg of total protein, after *in vitro* treatment with a compound of formula (I) or a pharmaceutical acceptable salt thereof is observed.

As used herein, the term "subject" refers to a warm-blooded animal such as a mammal, animal or human, in particular a human, who is afflicted with, or has the potential to be afflicted with one or more diseases and conditions described herein, and more particularly, in whom cancers cells both overproduce H₂O₂ in comparison to a control value as defined in claim 1 and have a level of GSH below 0,5 nmol for 25 000 cells. More particularly, said cancer cells are human cancer cells. Still particularly, cancer cells of said subject that overproduce H₂O₂ in comparison to a control value, more particularly that both overproduce H₂O₂ in comparison to a control value and have a level of GSH below 0,5 nmol for 25 000 cells are chemoresistant and/or radioresistant cancer cells. Even more particularly, cancer cells of said subject that overproduce H₂O₂ in comparison to a control value, more particularly that both overproduce H₂O₂ in comparison to a control value and have a level of GSH below 0,5 nmol for 25 000 cells, have also a MDA-adducts level above 75 ng per µg of total protein and/or a HNE-adducts level above 1 µg per µg of total protein after *in vitro* treatment with a compound of formula (I) or a pharmaceutical acceptable salt thereof.

The terms "overproduce", "overproduction", as used herein, describe a situation, where in a diseased cell or tissue, a compound is produced in overabundance compared to a corresponding control cell or tissue named control value.

In particular, the present invention thus relates to the compound for use according to the invention, wherein said subject is identified by measuring the H₂O₂ level and the GSH level in cancer cells of said subject. In one embodiment, said subject is also identified by measuring the MDA-adducts level and/or HNE-adducts level after an *in vitro* treatment with a compound of formula (I) according to the invention or a pharmaceutical acceptable salt thereof.

The level of H₂O₂ may be, for instance, measured by any method known to the skilled person, such as, for example, by determining the level of Relative Fluorescence Intensity, which can be done for example thanks to the Total ROS/superoxide detection kit (Enzo life science), in particular as measured on Appliskan fluorescence microplate reader (Thermo Scientific) (Ex/ Em= 488/520 nm and Ex/ Em= 550/610 nm).

In particular, the present invention thus relates to a compound for use according to the invention, wherein said H₂O₂ level is determined by quantifying the level of Fluorescence Intensity.

More particularly, it relates to a compound for use according to the invention, wherein said H₂O₂ level is higher than a value a value comprised between 2 000 and 400 000 Relative Fluorescence Intensity, for example between 10 000 and 100 000 Relative Fluorescence Intensity or 15 000 and 100 000 Relative Fluorescence Intensity, and more particularly a H₂O₂ level higher than 20 000 Relative Fluorescence Intensity, even more particularly higher than 21 598 Relative Fluorescence Intensity.

The skilled person will readily appreciate that any other parameter suitable for determining the H₂O₂ level of cells can be used in conjunction with the present invention.

Accordingly, any other methods known by the person skilled in the art to detect the level of H₂O₂ can be used without departing from the scope of the invention.

In particular, the present invention thus relates to the compound for use according to the invention, wherein said H₂O₂ level is determined by quantifying the level of Fluorescence Intensity thanks to the Total ROS/superoxide detection kit (Enzo life science), more particularly as measured on Appliskan fluorescence microplate reader (Thermo Scientific) (Ex/ Em= 488/520 nm and Ex/ Em= 550/610 nm).

If the method used in the experimental part is a determination of the level of H₂O₂ by mean fluorescence intensity, it is again stated that this method is nonexclusive and that the level of H₂O₂ can be determined by any other method available to the man skilled in the art.

The level of GSH is determined by any method available to the man skilled in the art. For example, the level of GSH is determined by luminescence, for example with the Promega GSH-Glo kit (Promega).

The skilled person will readily appreciate that any other parameter suitable for determining the GSH level of cells can be used in conjunction with the present invention.

Accordingly, any other methods known by the person skilled in the art to detect the level of GSH can be used without departing from the scope of the invention.

In particular, the present invention thus relates to the compound for use according to the invention, wherein said GSH level is determined by luminescence, more particularly with the Promega GSH-Glo kit (Promega).

The level of MDA-adducts is determined by any method available to the man skilled in the art. For example, the level of MDA-adducts is determined by immuno-monitoring, for example with OxiSelect™ MDA-adduct competitive ELISA kit (CELL BIOLABS).

The skilled person will readily appreciate that any other parameter suitable for determining the MDA-adducts level of cells can be used in conjunction with the present invention.

Accordingly, any other methods known by the person skilled in the art to detect the level of MDA-adducts can be used without departing from the scope of the invention.

In one embodiment, the level of MDA-adducts is thus measured after an *in vitro* treatment with a compound of formula (I) according to the invention or a pharmaceutical acceptable salt thereof.

The level of HNE-adducts is determined by any method available to the man skilled in the art. For example, the level of HNE-adducts is determined by immuno-monitoring, for example with OxiSelect™ HNE-adduct competitive ELISA kit (CELL BIOLABS).

The skilled person will readily appreciate that any other parameter suitable for determining the HNE-adducts level of cells can be used in conjunction with the present invention.

Accordingly, any other methods known by the person skilled in the art to detect the level of HNE-adducts can be used without departing from the scope of the invention.

In one embodiment, the level of HNE-adducts is thus measured after an *in vitro* treatment with a compound of formula (I) according to the invention or a pharmaceutical acceptable salt thereof.

As used herein, the term "sample" means a substance of biological origin. Examples of biological samples include, but are not limited to bodily fluids samples and biopsy. Bodily fluids include blood, urine, saliva or any other bodily secretion or derivative thereof. As used herein "blood" includes whole blood, plasma, serum, circulating epithelial cells, constituents, or any derivative of blood. The biological sample according to the invention may be obtained from the subject by any appropriate means of sampling known from the person skilled in the art.

For determining the level of H₂O₂, the level of GSH and/or the level of MDA-adducts and/or HNE adducts in cancer cells, the sample is in particular a biopsy of tumor, e.g. of a bladder tumor, brain tumor, breast tumor, melanoma tumor, multiple myeloma tumor, leukemia tumor, lymphoma tumor, prostate tumor, cervical tumor, stomach tumor, liver tumor, tongue tumor, ovarian tumor, pancreatic tumor, renal tumor, pleuramesothelomia tumor, osteosarcoma tumor, muscle tumor, lung tumor, kidney tumor, head and neck cancer tumor, colon tumor, blood tumor, tumors of the nervous central system and sarcoma tumor.

As regards to the comparison of level of H₂O₂, preferably the control value is measured in a sample of the same tissue origin as the sample of the cancer cells or the cancer sample, and more preferably in a sample of the same tissue origin as the sample of the cancer cells or the cancer sample of the same subject.

Preferably, the "control value" corresponds to the normal level of H₂O₂.

As intended herein a "normal level" of H₂O₂ means that the level of H₂O₂ in the sample is within the norm cut-off values for H₂O₂. The norm is dependent on the sample type and on the method used for measuring the level of H₂O₂ in the sample. In particular, the reference value of H₂O₂ may thus correspond to the absence of, or to a basal level of H₂O₂ in normal cells, preferably of the same tissue, and more preferably of the same tissue of the same subject or to the H₂O₂ value in cancer cells incubated with NAC preferably of the same tissue, and more preferably of the same tissue of the same subject.

NAC is an avid scavenger of hydroxy radicals (rate constant: 1.36x10¹⁰M⁻¹,s⁻¹) but which reacts slowly with hydrogen peroxide (rate constant:0.38M⁻¹, s⁻¹) and shows no reaction with superoxide anion (Aruoma et al ;Free Radic Biol Med , (1989) The antioxidant activity of N-acetyl cysteine: its reaction with hydrogen peroxide, hydroxy radical, superoxide anion, and hypochlorous acid:, 6, 593-597).

A level is considered to be statistically higher if the level of H₂O₂ in the cancer sample of the subject is increased to above the normal level of H₂O₂. In particular, the level of H₂O₂ is considered to be statistically higher if the level of H₂O₂ in the cancer sample of the patient is increased by order of at least 200 or 300 or 400 or 500 or 600% compared with the control value of level of H₂O₂.

In the same manner, a level is considered to be overproduced if the level of H₂O₂ in the cancer sample of the subject is increased to above the normal level of H₂O₂, more particularly by order of at least 200 or 300 or 400 or 500 or 600%, in particular by order of around 300% compared with the control value of level of H₂O₂.

A level is considered to be statistically higher if the level of H₂O₂ in the cancer sample of the subject is increased to above the level of H₂O₂ in cancer cells incubated with NAC. In particular, the level of H₂O₂ is considered to be statistically higher if the level of H₂O₂ in the cancer sample of the patient is increased by order of at least 200 or 300 or 400 or 500 or 600% compared with the value of level of H₂O₂ of the cancer sample incubated with NAC, preferably of the same tissue, and more preferably of the same tissue of the same subject.

In the same manner, a level is considered to be overproduced if the level of H₂O₂ in the cancer sample of the subject is increased to above the level of H₂O₂ in the cancer cells incubated with NAC, more particularly by order of at least 200 or 300 or 400 or 500 or 600% compared with the control value of level of H₂O₂.

The control value(s) may be determined as a single value or a range of values which is determined based on the level of H₂O₂ measured in a population of control cells i.e. normal cells, in particular in a population of normal cells of the same tissue origin as cancer cells, and more particularly from the same subject, or i.e. of cancer cells incubated with NAC, in particular in a population of cancer cells incubated with NAC, of the same tissue origin, and more particularly from the same subject.

The identification of a subject to be treated according to the invention relies on the comparison of the H₂O₂ level from a cancer cell sample with a control value as mentioned above. The control value may therein be a predetermined value or a value that is determined together with the measurement value.

Typically, the analysed population could be divided into quantiles based on the measured level of H₂O₂. The control value could be defined as the median, or the second tertile, or the second or third quartile, or the third or fourth quintile etc.

The control value of H₂O₂ may vary depending on the method used for measuring.

In one embodiment, when the cancer to treat is a leukaemia, the reference value is determined using the level of H₂O₂ in HL60 cells. The HL60 cell line is an established Human promyelocytic leukemia cell line well known by the man skilled in the art.

In particular, in said embodiment, the level of H₂O₂ is considered to be statistically higher or to be overproduced if "y" is higher than 2x/3, "x" being the level of H₂O₂ in the HL60 cells, and "y" being the level of H₂O₂ in the cancer sample of the subject.

The control value(s) may be determined as a single value or a range of values which is determined based on the level of H₂O₂ measured in a population of control cells i.e. HL60 cells.

Typically, the analysed population could be divided into quantiles based on the measured level of H₂O₂. The control value could be defined as the median, or the second tertile, or the second or third quartile, or the third or fourth quintile etc. The control value of H₂O₂ may vary depending on the method used for measuring.

The same method of comparison as the one described for HL60 cells when the cancer to teat is a leukaemia applies for the type of cancers mentioned in the table 1 below with the following corresponding cell lines as referenced values.

**Table 1**

| **Type of cancer** | **Referenced Cell line** |
|---|---|
| Bladder | HT-1197 |
| Blood | HL-60 |
| Brain | CGL-1 |
| Breast | MDA-MB-231 |
| Colon | Lovo |
| Head&Neck | Fadu |

The present invention further relates to a method of treatment of cancer in a subject, wherein cancer cells of said subject overproduce H₂O₂ in comparison to a control value, and in particular overproduce H₂O₂ in comparison to a control value and have a level of GSH below 0,5 nmol for 25 000 cells, said method comprising the administration of a therapeutically effective amount of a compound of formula (I) or a pharmaceutical acceptable salt thereof, in particular S-methyl 4-(dimethylamino)-4-methylpent-2-ynethioate, or a pharmaceutically acceptable salt thereof, more particularly 4-(Dimethylamino)-4-methyl-2-pentynethioic acid S-methyl ester fumarate.

In one embodiment, said cancer cells of said subject also have a MDA-adducts level above 75 ng per µg of total protein and/or a HNE-adducts level above 1 µg per µg of total protein, after *in vitro* treatment with a compound of formula (I) or a pharmaceutical acceptable salt thereof.

In one embodiment, the present invention also relates to a method for selecting a subject suffering from a cancer and who will most likely benefit from a treatment with a compound of formula (I): 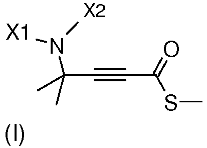 wherein X1 and X2, identical or different, are chosen among a C₁-C₇ alkyl group, a phenyl, a benzyl or X1 and X2 together with the nitrogen atom to which they are linked form an heterocycle, in particular a piperidine or a morpholine;
or a pharmaceutically acceptable salt thereof;
wherein said method comprises:
a) measuring the H₂O₂ level in a cancer cells sample of said subject;
b) comparing the resulting level of step a. with a control value; and/or
c) measuring the GSH level in a cancer cells sample of said subject;
wherein if:
- the H₂O₂ level of said cancer cells sample of said subject is higher than 200% of the control value, and
- the GSH level of said cancer cells sample of said subject is below 0.5 nmol per 25 000 cells, said method further comprises:
   d) treating said subject with a compound of formula (I) or a pharmaceutically acceptable salt thereof, in particular the S-methyl 4-(dimethylamino)-4-methylpent-2-ynethioate, or a pharmaceutically acceptable salt thereof.

In one embodiment, said method comprises:
a) measuring the H₂O₂ level in a cancer cells sample of said subject;
b) comparing the resulting level of step a. with a control value; and
c) measuring the GSH level in a cancer cells sample of said subject; and/or
c') measuring the MDA-adducts and/or HNE-adducts level after an *in vitro* treatment with a compound of formula (I) according to the invention or a pharmaceutical acceptable salt thereof in a cancer cells sample of said subject;
wherein if:
- the H₂O₂ level of said cancer cells sample of said subject is higher than 200% of the control value, and/or
- the GSH level of said cancer cells sample of said subject is below 0.5 nmol per 25 000 cells, and/or
- the MDA-adducts level is above 75 ng per µg of total protein and/or the HNE-adducts level is above 1µg per µg of protein
said method further comprises:
d) treating said subject with a compound of formula (I) or a pharmaceutically acceptable salt thereof, in particular the S-methyl 4-(dimethylamino)-4-methylpent-2-ynethioate, or a pharmaceutically acceptable salt thereof.

The compounds according to the invention may be prepared by a variety of synthetic routes. The reagents and starting materials are commercially available, or readily synthesized by well-known techniques by one of ordinary skill in the arts.

The identification of the subjects who are in need of treatment of herein-described diseases and conditions is conducted as above mentioned and is well within the ability and knowledge of one skilled in the art. A clinician skilled in the art can readily identify, by the above mentioned techniques, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances .

As used herein, a "therapeutically effective amount" refers to an amount which is effective in reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment and chronic use.

The amount of the compound according to the invention, which is required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient, and the route of administration.

The compound according to the invention can be formulated into pharmaceutical compositions by admixture with one or more pharmaceutically acceptable excipients.

As used herein, a "pharmaceutically acceptable excipient" refers to molecular entities and compositions that do not produce an adverse, allergic or other untoward reaction when administered to a mammal, especially a human, as appropriate. A pharmaceutically acceptable excipient refers to a non-toxic solid, semi-solid or liquid filler, diluent, encapsulating material or formulation auxiliary of any type.

Such compositions may be prepared for use in oral administration, particularly in the form of tablets or capsules, in particular orodispersible (lyoc) tablets; or parenteral administration, particularly in the form of liquid solutions, suspensions or emulsions.

It may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier. They can be administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition, as described hereinafter.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Liquid preparations, in particular for intravenous or oral administration, include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers, vegetable oils such as olive oil, and organic esters such as ethyl oleate. Aqueous carriers include mixtures of alcohols and water, hydrogels, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like.

Examples of modes of administration include parenteral e.g. subcutaneous, intramuscular, intravenous, intradermal, as well as oral administration. It includes in particular a formulation as a tablet for oral administration or as a powder for solution for injection for intravenous administration.

In the scope of the present invention, it has to be understood that "a compound of formula (I) or a pharmaceutically acceptable salt thereof, for use" is equivalent to "the use of a compound of formula (I) or a pharmaceutically acceptable salt thereof" and in particular that "a compound of formula (I) or a pharmaceutically acceptable salt thereof, for use in the treatment of" is equivalent to "the use of a compound of formula (I) or a pharmaceutically acceptable salt thereof, for the treatment of" and to "the use of a compound of formula (I), or a pharmaceutically acceptable salt thereof, for the manufacture of a medicament intended for the treatment of".

The present invention will be further illustrated by the following figures and examples.

### Figures

**Figure 1****:** efficacy of S-methyl 4-(dimethylamino)-4-methylpent-2-ynethioate (viability assay (resazurin, XTT or MTT) on various cancer cell lines
**Figure 2****:** H₂O₂/S-methyl 4-(dimethylamino)-4-methylpent-2-ynethioate IC₅₀ correlation: relationship between IC₅₀ of S-methyl 4-(dimethylamino)-4-methylpent-2-ynethioate in µM and the endogenous H₂O₂ activity levels in cancer cells
**Figure 3****:** H₂O₂/S-methyl 4-(dimethylamino)-4-methylpent-2-ynethioate IC₅₀ correlation: Determination of a cut-off
**Figure 4****:** H₂O₂/S-methyl 4-(dimethylamino)-4-methylpent-2-ynethioate IC₅₀ correlation: Studies by tissue origin
**Figure 5****:** Comparison of the H₂O₂ level of melanoma cells (cancer cells) and of melanocytes cells (corresponding normal cells)
**Figure 6****:** S-methyl 4-(dimethylamino)-4-methylpent-2-ynethioate effect in HL60 cancers cells that overproduce H₂O₂ and have or not a level of GSH below 0,5 nmol for 25 000 cells
**Figure 7****:** Inhibition of S-methyl 4-(dimethylamino)-4-methylpent-2-ynethioate efficacy by enhancing the level of GSH in cells having an overproduction of a H₂O₂
**Figure 8****:** S-methyl 4-(dimethylamino)-4-methylpent-2-ynethioate efficacy in Colo357 cells having an overproduction of a H₂O₂ and a level of GSH above 5 nmol for 25000 cells
**Figure 9****:** Total GSH level in nmol per 25000 cells observed in sensitive and resistant cells
**Figure 10****:** Quantification of MDA and HNE adducts in S-methyl 4-(dimethylamino)-4-methylpent-2-ynethioate sensitive cells (HL-60, NT2/D1) (A) and S-methyl 4-(dimethylamino)-4-methylpent-2-ynethioate resistant cells (MSC) treated with S-methyl 4-(dimethylamino)-4-methylpent-2-ynethioate 5 or 10 µmol.L⁻¹ during 24 hours (B)

Note: in all the figures mentioned above: DIMATE is given for S-methyl 4-(dimethylamino)-4-methylpent-2-ynethioate.

### Examples

### Example 1

### Material and methods

### Cell lines

A panel of 52 human tumor cells representing 10 tissue types has been selected to cover a broad set of different oncogenes and according to their response to different standard chemotherapeutics. Cells were obtained from the American Type Culture Collection (ATCC), the European Collection of Cell Cultures (ECACC) and from primary culture of cancer cells derived from patients' tumors (Research Institute of Vall d'Hebron (VHIR), Barcelone, Spain; Vall d'Hebron Institute of Oncology (VHIO), Barcelone, Spain; Oncotest, Freiburg, Germany; Oncodesign, Dijon, France; Universitá Degli Studi di Palermo, Oncology and Surgical Sciences, Palermo, Italy; and Institute of Predictive and Personalized Medicine of Cancer (IMPPC), Barcelona, Spain (See Table 2 for description of the tumor cell panel).

**Table 2**

| **Tissue** | **Characteristics** | **Cells** | Type **of cell line** |
|---|---|---|---|
| Bladder | | UM-UC-3 | established cell line |
| Bladder | | HT-1197 | established cell line |
| Bladder | | LB831-BLC | established cell line |
| Bladder | | RT112 | established cell line |
| Bladder | | T24 | established cell line |
| Blood | | HL-60 | established cell line |
| Blood | | OCI-AML2 | established cell line |
| Blood | | Raji | established cell line |
| Blood | | U-937 | established cell line |
| Blood | | Kasumi-1 | established cell line |
| Blood | | MOLM-14 | established cell line |
| Blood | | KG-1 | established cell line |
| Blood | | K-562/imatinib | established cell line |
| Blood | | THP-1 | established cell line |
| Brain | | CGL-1 | established cell line |
| Brain | | SK-N-AS | established cell line |
| Brain | | U-87 | established cell line |
| Brain | | BrGI2 | established cell line |
| Brain | | BrGI3 | established cell line |
| Brain | | BrGI6 | established cell line |
| Brain | | BrA1 | established cell line |
| Brain | | CGL-9 | established cell line |
| Breast | | SK-BR-3 | established cell line |
| Breast | | BT-20 | established cell line |
| Breast | | BT-474c | established cell line |
| Breast | | MCF-7 | established cell line |
| Breast | | MDA-MB-231 | established cell line |
| Breast | | MDA-MB-468 | established cell line |
| Breast | | BA-pt1102 | established cell line |
| Breast | | MBC-pt1106 | established cell line |
| Breast | | BA-pt1201 | established cell line |
| Breast | | BA-pt1202 | established cell line |
| Breast | | BA-pt1205 | established cell line |
| Cervix | | Hela | established cell line |
| Colon | | LoVo | established cell line |
| Colon | | SW620 | established cell line |
| Colon | | COLO-205 | established cell line |
| Colon | | HCT-15 | established cell line |
| Colon | | CRA07 | established cell line |
| Colon | | CRA11 | established cell line |
| Colon | | CRA13 | established cell line |
| Head & Neck | | LB1617-HNSCC | established cell line |
| Head & Neck | | Fadu | established cell line |
| Head & Neck | | KB | established cell line |
| Kidney | | Caki-1 | established cell line |
| Kidney | | A-498 | established cell line |
| Liver | | Hep G2 | established cell line |
| Liver | | Hep 3B2.1-7 | established cell line |
| Lung | | A-549 | established cell line |
| Lung | | A-549 | established cell line |
| Lung | | H-522 | established cell line |
| Lung | | PC-9 | established cell line |
| Lung | | HCC4006 | established cell line |
| Lung | | HCC2935 | established cell line |
| Lung | | H1975 | established cell line |
| Lung | | H1650 | established cell line |
| Lung | | H820 | established cell line |
| Lung | | H2935 | established cell line |
| Lung | | HCC4006 | established cell line |
| Lung | | HCC827 | established cell line |
| Lung | | H1299 | established cell line |
| Lung | | Hop62 | established cell line |
| Lung | | H522 | established cell line |
| Lung | | H23 | established cell line |
| Lung | | H460 | established cell line |
| Lung | | H441 | established cell line |
| Melanoma | | SKMEL-103 | established cell line |
| Melanoma | | SKMEL-147 | established cell line |
| Melanoma | | MLMN-9 | established cell line |
| Melanoma | | MLMN-10 | established cell line |
| Melanoma | | UACC-903 | established cell line |
| Melanoma | | SKMEL-28 | established cell line |
| Melanoma | | MBrM12 | established cell line |
| Muscle | | A-673 | established cell line |
| Normal | | HMVEC | established cell line |
| Normal | | HUV-EC-C | established cell line |
| Normal | | MRC-5 | established cell line |
| Normal | | HSC CD34+ | established cell line |
| Normal | | MSC | established cell line |
| Osteosarcoma | | OS-0411 | established cell line |
| Ovary | | PD-OVC-17 | established cell line |
| Ovary | | A2780 | established cell line |
| Ovary | | A2780/Cis | established cell line |
| Ovary | | IGROV-1 | established cell line |
| Ovary | | SK-OV-3 | established cell line |
| Ovary | | OVCAR-3 | established cell line |
| Ovary | | OVCAR-4 | established cell line |
| Ovary | | PD-OVC-11 | established cell line |
| Ovary | | PD-OVC-02 | established cell line |
| Ovary | | PD-OVC-05 | established cell line |
| Pancreas | | Capan-1 | established cell line |
| Pancreas | | Capan-2 | established cell line |
| Ovary | | PD-OVC-17 | established cell line |
| Pancreas | | MIA PaCa-2 | established cell line |
| Prostate | | 22Rv1 | established cell line |
| Prostate | | LNCap | established cell line |
| Prostate | | PC3 | established cell line |
| Prostate | | DU145 | established cell line |
| Stomach | | KATO III | established cell line |
| Bladder | | 1036 | PDC |
| Bladder | | 1218 | PDC |
| Bladder | | 1228 | PDC |
| Bladder | | 1258 | PDC |
| Bladder | | 1352 | PDC |
| Bladder | | 439 | PDC |
| Central Nervous System | | 498 | PDC |
| Cervix | | 1729 | PDC |
| Cervix | | 1783 | PDC |
| Cervix | | 2025 | PDC |
| Cervix | | 280 | PDC |
| Cervix | | 742 | PDC |
| Cervix | | 94 | PDC |
| Cervix | | 975 | PDC |
| Gastric | Asian | 3013 | PDC |
| Asian Gastric | Asian | 3044 | PDC |
| Asian Gastric | Asian | 3052 | PDC |
| Gastric | Caucasian | 1172 | PDC |
| Gastric | Caucasian | 214 | PDC |
| Gastric | Caucasian | 251 | PDC |
| Head and Neck; | Caucasian | 1842 | PDC |
| Lung | Adeno | 1041 | PDC |
| Lung | Adeno | 1584 | PDC |
| Lung | Adeno | 1647 | PDC |
| Lung | Adeno | 289 | PDC |
| Lung | Adeno | 526 | PDC |
| Lung | Adeno | 623 | PDC |
| Lung | Adeno | 629 | PDC |
| Lung | Adeno | 629 | PDC |
| Lung | Adeno | 923 | PDC |
| Lung | Adeno | 983 | PDC |
| Lung | Epidermoid | 1422 | PDC |
| Lung | Epidermoid | 397 | PDC |
| Lung | Large Cell | 1072 | PDC |
| Lung | Large Cell | 1121 | PDC |
| Lung | Large Cell | 1674 | PDC |
| Lung | Large Cell | 430 | PDC |
| Lung | Large Cell | 529 | PDC |
| Breast | | 1162 | PDC |
| Breast | | 1322 | PDC |
| Breast | | 1384 | PDC |
| Breast | | 583 | PDC |
| Breast | | 713 | PDC |
| Breast | | MX1 | established cell line |
| Melanoma | | 1765 | PDC |
| Melanoma | | 1792 | PDC |
| Melanoma | | 274 | PDC |
| Melanoma | | 276 | PDC |
| Melanoma | | 462 | PDC |
| Melanoma | | 520 | PDC |
| Melanoma | | 622 | PDC |
| Melanoma | | 672 | PDC |
| Ovarian | | 1023 | PDC |
| Ovarian | | 1353 | PDC |
| Ovarian | | 1544 | PDC |
| Ovarian | | 899 | PDC |
| Pancreas | | 1872 | PDC |
| Pancreas | | 1900 | PDC |
| Pancreas | | 1986 | PDC |
| Pancreas | | 2033 | PDC |
| Pancreas | | 2082 | PDC |
| Pancreas | | 2116 | PDC |
| Pancreas | | 546 | PDC |
| Prostate | | DU-145 | established cell line |
| Prostate | | MRI-H-1579 | established cell line |
| Prostate | | PC-3M | established cell line |
| Pleuramesothelioma | | 1752 | PDC |
| Pleuramesothelioma | | 541 | PDC |
| Renal | | 1114 | PDC |
| Renal | | 1183 | PDC |
| Renal | | 1393 | PDC |
| Renal | | 486 | PDC |
| Renal | | 616 | PDC |
| Sarcoma | | 1937 | PDC |
| Colon | | CXF 1297 | PDC |
| Colon | | CXF 243 | PDC |
| Colon | | CXF 280 | PDC |
| Colon | | CXF 647 | PDC |
| Colon | | CXF 676 | PDC |
| Gastric | Asian | GXA 3011 | PDC |
| Gastric | Asian | GXA 3023 | PDC |
| Gastric | Caucasian | GXF 97 | PDC |
| Head & Neck | Caucasian | HNXF 536 | PDC |
| Head & Neck | Caucasian | HNXF 908 | PDC |
| Lung | Adeno | LXFA 400 | PDC |
| Lung | Adeno | LXFA 586 | PDC |
| Breast | | MAXF 449 | PDC |
| Breast | | MAXF 508 | PDC |
| Breast | | MAXF 583 | PDC |
| Breast | | MAXF 713 | PDC |
| Breast | | MAXF MX1 | PDC |
| Melanoma | | MEXF 1829 | PDC |
| Melanoma | | MEXF 989 | PDC |
| Ovary | | OVXF 1023 | PDC |
| Ovary | | OVXF 1353 | PDC |
| Ovary | | OVXF 1544 | PDC |
| Ovary | | OVXF 899 | PDC |
| Pancreas | | PAXF 1998 | PDC |
| Pancreas | | PAXF 2005 | PDC |
| Pancreas | | PAXF 2045 | PDC |
| Pancreas | | PAXF 2046 | PDC |
| Pancreas | | PAXF 2053 | PDC |
| Pancreas | | PAXF 2059 | PDC |
| Pancreas | | PAXF 2094 | PDC |
| Pancreas | | PAXF 546 | PDC |
| Renal | | RXF 1220 | PDC |
| Renal | | RXF 1781 | PDC |
| Renal | | RXF 631 | PDC |
| Skin | | SXFS 117 | PDC |

All cells were cultivated in appropriate media according to supplier recommendations.

### Treatment with S-methyl 4-(dimethylamino)-4-methylpent-2-vnethioate

Cells were seeded into 96-well cell culture plates at concentrations required to ensure approximately 80% confluence in control (untreated cells) at the end of experiment (0.5x 10⁴ - 5x 10⁴ cell/well). The sensitivity towards S-methyl 4-(dimethylamino)-4-methylpent-2-ynethioate was determined using different concentrations of the drug (from 0.01 to 100 µM). After 48 hours, the growth-inhibitory effect of the drug was analyzed using Alamar blue, according to manufactures instructions. The Alamar Blue assay was used based on earlier observations: similar results were found with Alamar blue, tetrazolium reduction (XTT assay). To ensure good data quality and to minimize impact of pipetting errors, each particular drug concentration was assessed based on mean fluorescence intensity from 8 separate wells. The drug response was quantified by the half maximal inhibitory concentration (IC₅₀) for each particular cell line, and determined by non-linear regression analysis of log-dose/response curves. Cut-off value for resistance to S-methyl 4-(dimethylamino)-4-methylpent-2-ynethioate was determined statistically (> 2 S.D. above the IC₅₀ geometric mean). The *in-vitro* threshold value for hypersensitivity to the drug has been defined as < IC₅₀ geometric mean.

### 3D Clonogenic assay, 96 well format

The clonogenic assay test was carried out in 96 well plate format. For each test a frozen aliquot of tumor cells prepared from tumor xenografts was thawed and assay plates were prepared as follows: each test well contains 3 layers of equal volume, 2 layers of semi-solid medium (bottom and top layer), and one layer of medium supernatant, with or without test compound. The bottom layer consists of 0.05 mL/well Iscove's Modified Dulbecco's Medium (Invitrogen), supplemented with 20% (v/v) fetal calf serum (Sigma), 0.01% (w/v) gentamicin (Invitrogen) and 0.75% (w/v) agar (BD Biosciences). Tumor cells were added to 0.05 mL of the same culture medium supplemented with 0.4% (w/v) agar and plated onto the bottom layer. After 24 h test compounds were added after serial dilution in DMSO and transfer in cell culture medium, and left on the cells for the duration of the experiment (continuous exposure, 0.05 mL drug overlay). Every dish includes six untreated control wells and drug-treated groups in duplicate at 10 concentrations. Cultures were incubated at 37 °C and 7.5% CO₂ in a humidified atmosphere for 8 to 13 days and monitored closely for colony growth using an inverted microscope. Within this period, ex *vivo* tumor growth leads to the formation of colonies with a diameter superior to 50 µm. At the time of maximum colony formation, counts were performed with an automatic image analysis system. 24h prior to evaluation, vital colonies were stained with a sterile aqueous solution of 2-(4-iodophenyl)-3-(4-nitrophenyl)-5-phenyltetrazolium chloride (1 mg/mL, 100 µL/well). Sigmoidal concentration-response curves were fitted to the data points obtained for each tumor model using 4 parameter non-linear curve fit (Oncotest Warehouse Software). IC₅₀ values were reported as relative IC₅₀ values, being the concentration of test compound that give a response (inhibition of colony formation/viability) half way between the top and bottom plateau of the sigmoidal concentration-response curve (inflection point of the curve), or as absolute IC₅₀ values, being the concentration of test compound at the intersection of the concentration-response curves with T/C = 50%. For calculation of mean IC₅₀ values the geometric mean was used. Results were presented as mean graph plots or heat maps (individual IC₅₀ values relative to the geometric mean IC₅₀ value) over all tumor models as tested.

### Measurement of H₂O₂ production

Intracellular H₂O₂ production in live cells was measured using Total ROS/superoxide detection kit (Enzo life science), following the manufacturer's instructions. The assay uses specific H₂O₂/RNS probes that upon reaction with H₂O₂ and RNS species were oxidized rapidly to highly fluorescent compounds. Fluorescence intensity is proportional to the total H₂O₂/RNS levels within the sample. The experimental tests were performed using untreated cells, or cells treated with S-methyl 4-(dimethylamino)-4-methylpent-2-ynethioate (1-5uM), vehicle HEPES, N-acetyl-L-cysteine (NAC) (10mM). The day before the analysis, cells were seeded in 96-well black/clear bottom plates at a density of 2x 10⁴ in culture media according to cell suppliers instructions, supplemented with FBS (10% v/v), 10 units of penicillin/ml and 100 mg of streptomycin. Cells were kept at 37°C in a humid atmosphere of 95% air: 5% CO₂ overnight. On the day of the analysis, cells media was replaced by 100 µL of phenol free-media supplemented as above, without any treatment, with S-methyl 4-(dimethylamino)-4-methylpent-2-ynethioate for 6 hours, vehicle HEPES for 6 hours, or with NAC for 20 min. After the incubation period, wells were loaded with 100 µL of H₂O₂ Detection Solution, containing the respective treatment agent or controls and incubated for 60 min at 37°C in the dark. Several wells were left without cells for the background fluorescence control measurements. Plates were read using fluorescence microplate reader (ex= 560 nm, Em=600). Data were expressed as the change in arbitrary fluorescence units produced from equal amounts of cells and normalized to total protein input.

### Measurement of GSH production

Cells were seeded in 96-well black walled plate (5 × 10⁴ cells/mL) and incubated overnight for attachment. The cells were treated either with vehicle, DMSO, BCNU 100µM, S-methyl 4-(dimethylamino)-4-methylpent-2-ynethioate 20µM, for 24 hours. The total glutathione was measured according to the instructions of the kit (GSH-Glo™, Promega) and results were obtained for 25 000 cells.

### Quantitative determination of HNE-protein and MDA/protein adducts by ELISA

The formation of HNE-adducts and MDA-adducts was quantified with the Oxiselect HNE Adduct Elisa kit (Cell Biolabs, San Diego, CA) and the OxiSelect MDA Adduct ELISA Kit (Cell Biolabs), respectively. Briefly, after a treatment with S-methyl 4-(dimethylamino)-4-methylpent-2-ynethioate during 24 hours, cells were lysed by sonication in reducing SDS Sample Buffer. Homogenates were diluted to 10 µg protein/mL and adsorbed in 96-well protein binding plates by incubation at 37°C for at least 2 hours. Wells were washed twice with PBS and incubated for an additional 2 hours at room temperature on an orbital shaker. Following three washes in PBS, 100 µL of anti-HNE antibody or anti-MDA antibody were added to the wells and incubated for 1 hour at room temperature. Subsequently, goat anti-rabbit secondary antibody-HRP conjugate (diluted 1/1000 with the assay diluent) was added and incubation continued for 1 hour. Wells were washed five times in PBS and HRP-substrate was added. Reaction was stopped with an acidic solution, and absorbance read on a microplate reader at 450nm. The level of HNE-adducts and of MDA-adducts was determined by comparison with a standard curve prepared from HNE-BSA and MDA-BSA standards supplied by the manufacturer.

### Induction of resistance to S-methyl 4-(dimethylamino)-4-methylpent-2-ynethioate treated cells.

To revert S-methyl 4-(dimethylamino)-4-methylpent-2-ynethioate -induced cell death, drug sensitive cells HL-60 were exposed to S-methyl 4-(dimethylamino)-4-methylpent-2-ynethioate (10µM) in the presence of the reduced glutathione analog Glutathione Monoethyl Ester (GSH-MEE) at concentrations 10 mM or S-methyl 4-(dimethylamino)-4-methylpent-2-ynethioate in combination. HL-60 were seeded, into 96-wells plate, at a concentration of 5 x10⁴ cells/well in 100µL of complemented medium supplemented with Glutathione Monoethyl Ester (GSH-MEE) 10 mM. After 90 minutes incubation, cells were washed with PBS and cells were incubated using different concentrations of the drug (from 0.01 to 100 µM). After 24h and 48h cell viability was measure using the Rezasurin assay as described above.

### Statistical Analysis

Values are expressed as mean ± SD or frequencies and proportions. Differences between groups were determined by unpaired t test, Chi-square, Fisher's exact test or ANOVA, where appropriate. P <0.05 was considered statistically significant. Analysis was performed using GraphPad prism version 5.0 (GraphPad software, San Diego California USA) and JMP software version 12.01 (SAS Institute Inc. North Carolina USA).

### Results

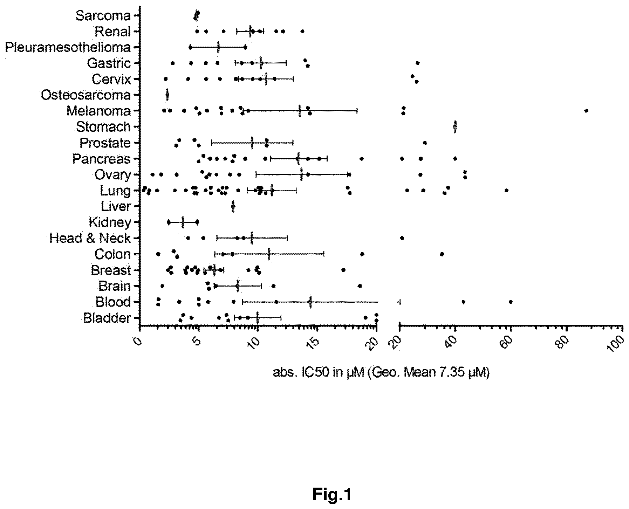

The results obtained are shown in Figures 1 to 10.

### Efficacy of S-methyl 4-(dimethylamino)-4-methylpent-2-ynethioate in vitro

As shown in Figure 1, S-methyl 4-(dimethylamino)-4-methylpent-2-ynethioate is effective in a wide variety of cancer cell lines which overproduce H₂O₂ (Viability assay-resazurin, XTT or MTT as explained above).

### H₂O₂/S-methyl 4-(dimethylamino)-4-methylpent-2-ynethioate IC₅₀ correlation

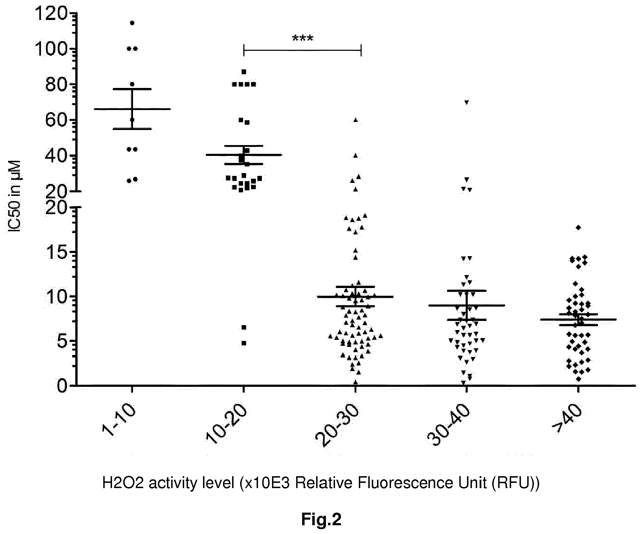

Figure 2 shows that a correlation is observed between high H₂O₂ activity and low IC₅ₒ (monitoring by Total ROS/superoxide detection kit as mentioned above).

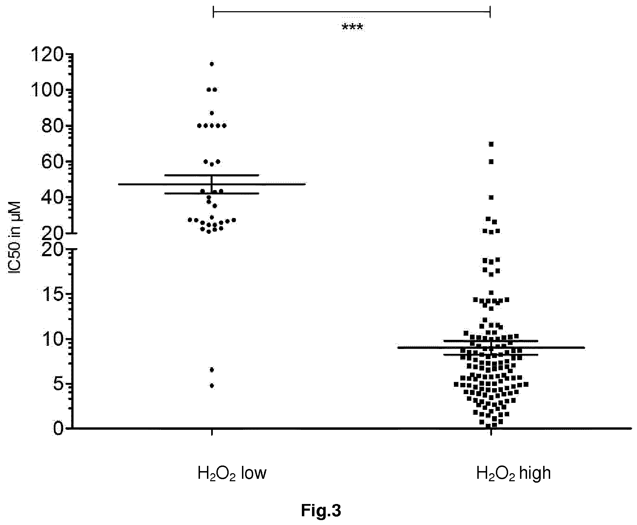

In addition, Figure 3 shows that by separating the cells in two parts with a cut-off (20 000 Relative Fluorescence Intensity), a distinction of S-methyl 4-(dimethylamino)-4-methylpent-2-ynethioate sensitivity to H₂O₂ high from H₂O₂ low is revealed (Pvalue < 0.05).

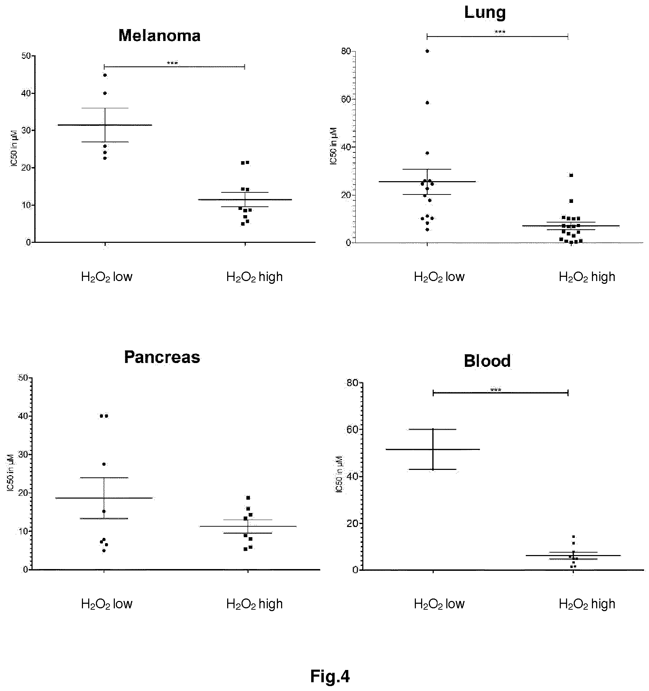

In Figure 4, the correlation between high H₂O₂ activity and low IC₅₀ is illustrated by tissue origin.

In addition, to control the selectivity of the S-methyl 4-(dimethylamino)-4-methylpent-2-ynethioate toward cancer cells, the compound according to the invention was also tested on normal cells by using the same method as the one described above. The results show that the compound according to the invention has no effect on normal cells as shown in Table 3 below.

**Table 3**

| S-methyl 4-(dimethylamino)-4-methylpent-2-ynethioate in normal human primary cells | | |
|---|---|---|
| **Cell line** | **Description** | ***IC50 (uM)*** |
| hMCSs-BM | Human mesenchymal stem cells from bone marrow | >50 |
| huvec | Human umbilical vein endothelial cells | >50 |
| BEAS-2B | Human bronchial epithelial cells | >50 |
| HPF | Human Pulmonary fibroblasts | >100 |
| NHEM | Normal Human epidermal melanocytes | >100 |

In addition, other compounds according to the invention were tested by following the above mentioned material and method on the cell line DU145 (prostatic cancer line) and the cell line HL60 (leukemia) mentioned in Table 2.

The results regarding the efficacy of those compounds are provided in the Table 4 below.

**Table 4**

| IUPAC Name | Formula | | IC₅₀(D U145) | IC₅₀(HL -60) |
|---|---|---|---|---|
| S-methyl 4-methyl-4-(piperidin-1-yl)pent-2-ynethioate | 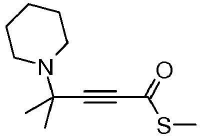 | Formula Weight : 225,4 | 22 µM | 7.211 µM |
| | | Formula : C₁₂H₁₉NOS | | |
| S-methyl 4-[benzyl(methyl)amino]-4-methylpent-2-ynethioate | 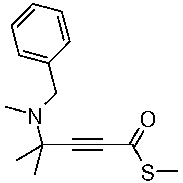 | Formula Weight : 261,4 | 1.4 µM | 10.408 µM |
| | | Formula : C₁₅H₁₉NOS | | |
| S-methyl 4-methyl-4-[methyl(phenyl)amino]pen t-2-ynethioate | 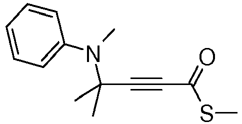 | Formula Weight : 247,4 | 12.5 µM | 24.8 µM |
| | | Formula : C₁₄H₁₇NOS | | |
| S-methyl 4-methyl-4-(morpholin-4-yl)pent-2-ynethioate | 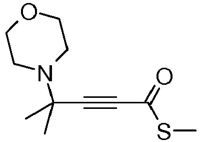 | Formula Weight : 227,3 | 9.3 µM | 30 µM |
| | | Formula : C₁₁H₁₇NO₂S | | |
| S-methyl 4-(dimethylamino)-4-methylpent-2-ynethioate | 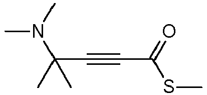 | Formula Weight : 185.28 | 7.2 µM | 7.6 µM |
| | | Fumarate Salt: 301.2 | | |
| | | Formula : C₉H₁₅NOS | | |

### Correlation between H₂O₂ level and GSH level

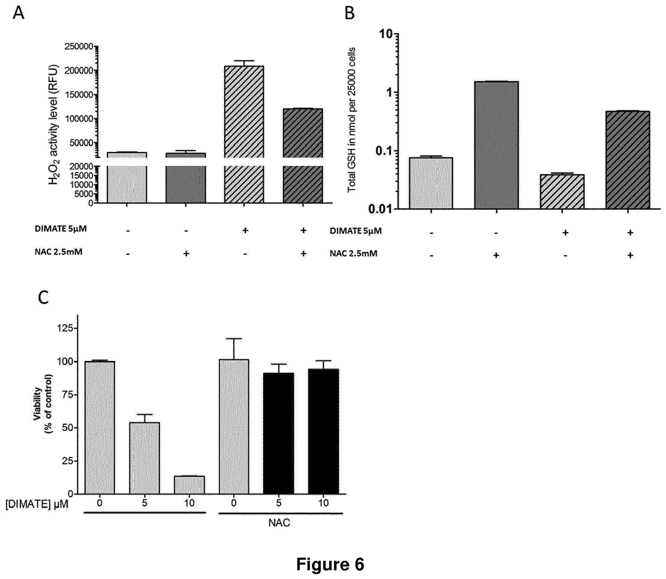

The Figure 6 shows S-methyl 4-(dimethylamino)-4-methylpent-2-ynethioate effect in HL60 cancers cells that overproduce H₂O₂ and have or not a level of GSH below 0,5 nmol for 25 000 cells.

In particular, Figure 6(A) shows that the H₂O₂ activity level in Relative Fluorescence Unit (RFU) was increased after treatment with S-methyl 4-(dimethylamino)-4-methylpent-2-ynethioate 5 µM for 24 hours. However, no H₂O₂ increase was observed using a treatment of S-methyl 4-(dimethylamino)-4-methylpent-2-ynethioate in addition with 2.5mM N-acetylCysteine (NAC). In Figure 6(B), the amount of GSH level was significantly increased in HL-60 cells treated with 2.5mM NAC and the cells were thus overproducing H₂O₂ and have a level of GSH above 0,5 nmol for 25 000 cells.

Figure 6 (C) shows the viability of HL-60 cells treated with S-methyl 4-(dimethylamino)-4-methylpent-2-ynethioate (5, 10µmol.L-1) and/or 2.5mM NAC, and that the inhibition of efficacy of S-methyl 4-(dimethylamino)-4-methylpent-2-ynethioate was significantly observed in HL-60 cells treated with NAC.

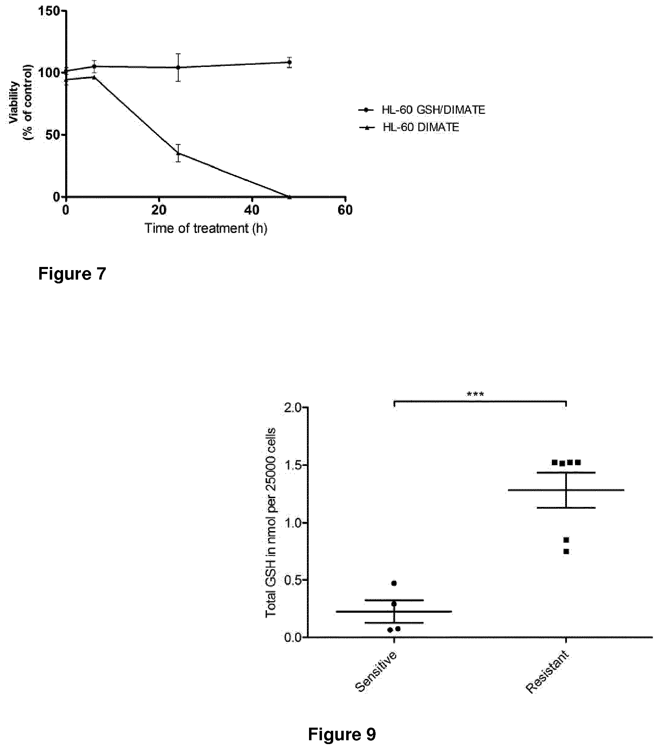

In addition, Figure 7 shows that after a pre-treatment of 10mM GSH-MEE, a Glutathione reduced ethyl ester (GSH-MEE) being a membrane/lipid permeable derivative of GSH that may be used to partially supplement the GSH supply, treatment of HL-60 cells using GSH-MEE inhibits the cytotoxic effect of S-methyl 4-(dimethylamino)-4-methylpent-2-ynethioate. By adding more GSH in cells, the status of cells became: overproduction H₂O₂ and level of GSH above 0,5 nmol for 25 000 cells, and S-methyl 4-(dimethylamino)-4-methylpent-2-ynethioate is no more effective.

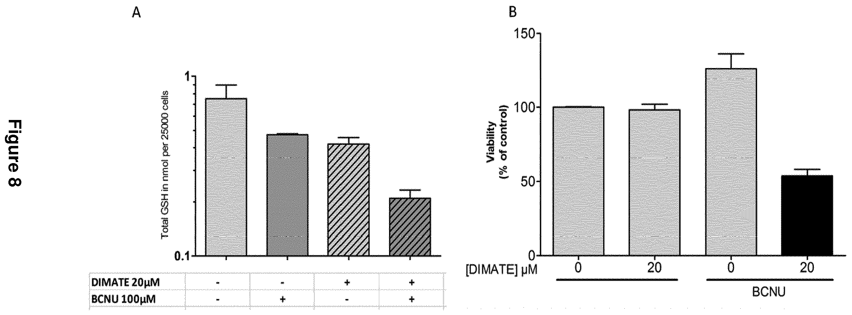

Furthermore, to confirm these results, Figure 8 shows that (A) the treatment of Colo357 cells with 100µM carmustine (BCNU) allows a 50% decrease of the GSH level and to change the status of cells in: overproduction H₂O₂ and level of GSH below 0,5 nmol for 25 000 cells and that as a consequence (B) S-methyl 4-(dimethylamino)-4-methylpent-2-ynethioate was effective on these cells, decreasing the viability of 50%.

In Figure 9, the total GSH level in nmol per 25000 cells is observed in sensitive and resistant cells. A significative difference (***, p-value<0.001) was observed. Using a threshold of 0.5 nmol per 25000 cells for distinction of GSH high from GSH low cells, 100% of sensitive cells are GSH low and 100% of resistant cells are GSH high.

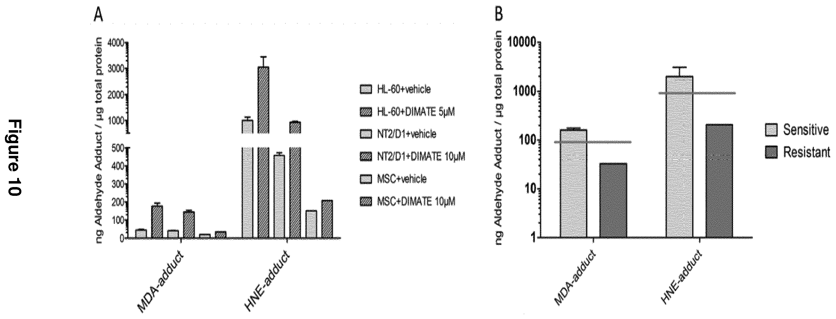

Figure 10 shows the quantification of MDA and HNE adducts in S-methyl 4-(dimethylamino)-4-methylpent-2-ynethioate sensitive cells (HL-60, NT2/D1) (A) and S-methyl 4-(dimethylamino)-4-methylpent-2-ynethioate resistant cells (MSC) treated with S-methyl 4-(dimethylamino)-4-methylpent-2-ynethioate 5 or 10 µmol.L⁻ during 24 hours (B). For MDA-adducts, in sensitive S-methyl 4-(dimethylamino)-4-methylpent-2-ynethioate cells, the threshold is above 100 ng per µg of total protein, and in resistant cells, the threshold is below this threshold. For HNE-adducts, the same observation can be made with a threshold of 1 µg per µg of total protein.

### Example 2:

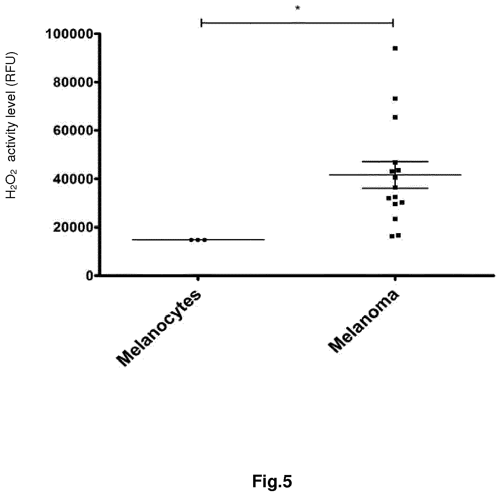

The H₂O₂ level from a melanoma cells sample is compared to a control value to determine if these cells would be eligible to a treatment with the compounds according to the invention.
The H₂O₂ level from human melanoma cells (obtained as mentioned in example 1) is thus measured as well as the H₂O₂ level from the corresponding normal cells, here melanocytes (Normal human epithelial melanocytes from juvenile foreskin, Promocell (NHEM, pool) cultivated with Melanocyte growth medium M2 of Promocell).
The two samples are subjected to determination of the H₂O₂ level using the Total ROS/superoxide detection kit (Enzo life science). Relative Fluorescence Intensities of both samples are measured on Appliskan fluorescence microplate reader (Thermo Scientific) (Ex/ Em= 488/520 nm and Ex/ Em= 550/610 nm).
As can be seen from Figure 5, the H₂O₂ level of the cancer cells sample exceeds the H₂O₂ level of the control sample (i.e. normal cells) by 280%.

These cancer cells are therefore eligible to treatment with the compounds according to the invention, and in particular with 4-(Dimethylamino)-4-methyl-2-pentynethioic acid S-methyl ester fumarate, if the level of GSH is at the same time below 5 nmol for 25000 cells.

## Claims

1. A compound of formula (I): 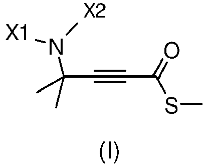 wherein X1 and X2, identical or different, are chosen among a C₁-C₇ alkyl group, a phenyl, a benzyl or X1 and X2 together with the nitrogen atom to which they are linked form an heterocycle, in particular a piperidine or a morpholine;
or a pharmaceutical acceptable salt thereof;
for use for the treatment of cancer in a subject,
wherein cancer cells of said subject: - have an increased level of H₂O₂ of at least 200 % compared with the basal level of H₂O₂ in normal cells originating from the same tissue as the cancer cells; and
- have a level of GSH below 0,5 nmol for 25 000 cells, and wherein said subject is identified by measuring the H₂O₂ level and the GSH level in cancer cells of said subject.

2. A compound of formula (I) or a pharmaceutically acceptable salt thereof for use according to claim 1, wherein said H₂O₂ level is determined by quantifying the level of Fluorescence Intensity.

3. A compound of formula (I) or a pharmaceutically acceptable salt thereof for use according to claim 2, wherein said H₂O₂ level is higher than 20000 Relative Fluorescence Intensity.

4. A compound of formula (I) or a pharmaceutically acceptable salt thereof for use according to any of claims 1 to 2, wherein cancer cells of said subject have also a MDA-adducts level above 75 ng per µg of total protein and/or a HNE-adducts level above 1 µg per µg of total protein, after *in vitro* treatment with a compound of formula (I) or a pharmaceutical acceptable salt thereof.

5. A method for selecting a subject suffering from a cancer and who will most likely benefit from a treatment with a compound of formula (I): 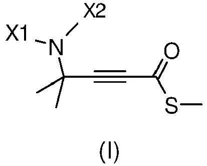 wherein X1 and X2, identical or different, are chosen among a C₁-C₇ alkyl group, a phenyl, a benzyl or X1 and X2 together with the nitrogen atom to which they are linked form an heterocycle, in particular a piperidine or a morpholine;
or a pharmaceutically acceptable salt thereof;
wherein said method comprises:
a. measuring the H₂O₂ level in a cancer cells sample of said subject;
b. comparing the resulting level of step a. with the basal level of H₂O₂ in normal cells originating from the same tissue as the cancer cells₂; and
measuring the GSH level in a cancer cells sample of said subject;
wherein:
- an increased level of H₂O₂ of at least 200 % compared with the basal level of H₂O₂ in normal cells originating from the same tissue as the cancer cells, and
- a GSH level of said cancer cells sample of said subject below 0,5 nmol for 25 000 cells,
indicates that the subject is likely to benefit from a treatment with said compound or a pharmaceutically acceptable salt thereof.

6. Method according to claim 5, wherein said method comprises:
a) measuring the H₂O₂ level in a cancer cells sample of said subject;
b) comparing the resulting level of step a. with the basal level of H₂O₂ in normal cells originating from the same tissue as the cancer cells;
c) measuring the GSH level in a cancer cells sample of said subject; and
d) measuring the MDA-adducts and/or HNE-adducts level after an *in vitro* treatment with a compound of formula (I) or a pharmaceutical acceptable salt thereof in a cancer cells sample of said subject;
wherein:
- an increased level of H₂O₂ of at least 200 % compared with the basal level of H₂O₂ in normal cells originating from the same tissue as the cancer cells,
- a GSH level of said cancer cells sample of said subject below 0.5 nmol per 25 000 cells, and
- a MDA-adducts level above 75 ng per µg of total protein and/or a HNE-adducts level above 1µg per µg of protein,
indicates that the subject is likely to benefit from a treatment with said compound or a pharmaceutically acceptable salt thereof.

7. A method according to any of claims 5 or 6, wherein a H₂O₂ level higher than 20000 Relative Fluorescence Intensity, indicates that the subject is likely to benefit from a treatment with a compound of formula (I), or a pharmaceutically acceptable salt thereof.

8. A compound of formula (I) or a pharmaceutically acceptable salt thereof for use according to any of claims 1 to 4 or a method according to any of claims 5 to 7, wherein the GSH level is determined by luminescence.

9. A compound of formula (I) or a pharmaceutically acceptable salt thereof for use according to claim 4 or a method according to any of claims 6 to 8 , wherein the MDA-adducts level and/or the HNE-adducts level is determined by immuno-monitoring.

10. A compound of formula (I) or a pharmaceutically acceptable salt thereof for use according to any one of claims 1 to 4 and 8 to 9 or a method according to any one of claims 5 to 9, wherein the cancer is chosen from bladder cancer, brain tumors, breast cancer, melanoma, multiple myeloma, leukemia, lymphoma, prostate cancer, cervical cancer, stomach cancer, liver cancer, tongue cancer, ovarian cancer, pancreatic cancer, renal cancer, pleuramesothelomia, osteosarcoma, muscle cancer, lung cancer, kidney cancer, head and neck cancer, colon cancer, blood cancer, cancers of the nervous central system and sarcoma.

11. A compound for use according to any one of claims 1 to 4 and 8 to 10 or a method according to any one of claims 5 to 10, wherein in said compound of formula (I), X1 and X2, identical or different, are chosen among a methyl, a phenyl, a benzyl, at least one of X1 or X2 being a methyl, or X1 and X2 together with the nitrogen atom to which they are linked form a piperidine or a morpholine.

12. A compound for use according to any one of claims 1 to 4 and 8 to 11 or a method according to any one of claims 5 to 11, wherein the compound of formula (I) is chosen from:
- S-methyl 4-methyl-4-(piperidin-1-yl)pent-2-ynethioate;
- S-methyl 4-[benzyl(methyl)amino]-4-methylpent-2-ynethioate;
- S-methyl 4-methyl-4-[methyl(phenyl)amino]pent-2-ynethioate;
- S-methyl 4-methyl-4-(morpholin-4-yl)pent-2-ynethioate; and
- S-methyl 4-(dimethylamino)-4-methylpent-2-ynethioate.

13. A compound for use according to any one of claims 1 to 4 and 8 to 12 or a method according to any one of claims 5 to 12, wherein said compound of formula (I) is the S-methyl 4-(dimethylamino)-4-methylpent-2-ynethioate or a pharmaceutical acceptable salt thereof.

14. A compound for use according to claim 13 or a method according to claim 13, wherein said pharmaceutical acceptable salt is 4-(Dimethylamino)-4-methyl-2-pentynethioic acid S-methyl ester fumarate.

## Patentansprüche

1. Verbindung der Formel (I): 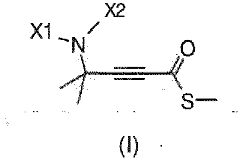 wobei X1 und X2, identisch oder unterschiedlich, ausgewählt sind aus einer C1-C7-Alkylgruppe, einem Phenyl, einem Benzyl, oder X1 und X2 bilden zusammen mit dem Stickstoffatom, an das sie gebunden sind, einen Heterozyklus, insbesondere ein Piperidin oder ein Morpholin;
oder ein pharmazeutisch annehmbares Salz davon;
zur Verwendung zur Behandlung von Krebs in einer Person/einem Tier,
wobei Krebszellen der Person/des Tiers:
- einen erhöhten H₂O₂-Spiegel von mindestens 200 % im Vergleich zum basalen H₂O₂-Spiegel in normalen Zellen aufweisen, die aus demselben Gewebe wie die Krebszellen stammen; und
- einen GSH-Spiegel von unter 0,5 nmol pro 25.000 Zellen haben, und wobei die Person/das Tier durch Messen des H₂O₂-Spiegels und des GSH-Spiegels in Krebszellen der Person/des Tiers identifiziert wird.

2. Verbindung der Formel (I), oder ein pharmazeutisch annehmbares Salz davon, zur Verwendung nach Anspruch 1, wobei der H₂O₂-Spiegel durch Quantifizierung des Fluoreszenzintensitätsniveaus bestimmt wird.

3. Verbindung der Formel (I), oder ein pharmazeutisch annehmbares Salz davon, zur Verwendung nach Anspruch 2, wobei der H₂O₂-Spiegel höher als 20.000 Relative Fluoreszenzintensität ist.

4. Verbindung der Formel (I), oder ein pharmazeutisch annehmbares Salz davon, zur Verwendung nach einem der Ansprüche 1 bis 2, wobei Krebszellen der Person/des Tiers nach einer *in vitro*-Behandlung mit einer Verbindung der Formel (I) oder einem pharmazeutisch annehmbaren Salz davon auch einen Spiegel an MDA-Addukten über 75 ng pro pg Gesamtprotein und/oder einen Spiegel an HNE-Addukten über 1 pg pro pg Gesamtprotein aufweisen.

5. Verfahren zur Auswahl einer Person/eines Tiers, die/das an einem Krebs leidet und die/das höchstwahrscheinlich von einer Behandlung mit einer Verbindung der Formel (I) profitieren wird: 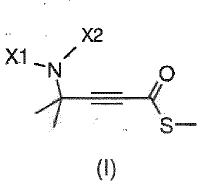 wobei X1 und X2, identisch oder unterschiedlich, ausgewählt sind aus einer C1-C7-Alkylgruppe, einem Phenyl, einem Benzyl, oder X1 und X2 bilden zusammen mit dem Stickstoffatom, an das sie gebunden sind, einen Heterozyklus, insbesondere ein Piperidin oder ein Morpholin;
oder ein pharmazeutisch annehmbares Salz davon;
wobei das Verfahren umfasst:
a. Messen des H₂O₂-Spiegels in einer Krebszellenprobe der Person/des Tiers;
b. Vergleichen des resultierenden Spiegels von Schritt a. mit dem basalen H₂O₂-Spiegel in normalen Zellen, die aus dem gleichen Gewebe wie die Krebszellen stammen; und
Messen des GSH-Spiegels in einer Krebszellenprobe der Person/des Tiers;
wobei
- ein erhöhter H₂O₂-Spiegel von mindestens 200 % im Vergleich zum basalen H₂O₂-Spiegel in normalen Zellen, die aus demselben Gewebe wie die Krebszellen stammen, und
- ein GSH-Spiegel in der Krebszellenprobe der Person/des Tiers von unter 0,5 nmol pro 25.000 Zellen
darauf hinweist, dass die Person/das Tier wahrscheinlich von einer Behandlung mit dieser Verbindung, oder einem pharmazeutisch annehmbaren Salz davon, profitiert.

6. Verfahren nach Anspruch 5, wobei das Verfahren umfasst:
a) Messen des H₂O₂-Spiegels in einer Krebszellenprobe der Person/des Tiers;
b) Vergleichen des resultierenden Spiegels von Schritt a. mit dem basalen H₂O₂-Spiegel in normalen Zellen, die aus dem gleichen Gewebe wie die Krebszellen stammen;
c) Messen des GSH-Spiegels in einer Krebszellenprobe der Person/des Tiers; und
d) Messen des Spiegels an MDA-Addukten und/oder HNE-Addukten nach einer *In*-*vitro*-Behandlung mit einer Verbindung der Formel (I), oder einem pharmazeutisch annehmbaren Salz davon, in einer Krebszellenprobe der Person/des Tiers;
wobei
- ein erhöhter H₂O₂-Spiegel von mindestens 200 % im Vergleich zum basalen H₂O₂-Spiegel in normalen Zellen, die aus demselben Gewebe wie die Krebszellen stammen,
- ein GSH-Spiegel in der Krebszellenprobe der Person/des Tiers von unter 0,5 nmol pro 25.000 Zellen und
- ein Spiegel an MDA-Addukten über 75 ng pro pg Gesamtprotein und/oder einen Spiegel an HNE-Addukten über 1 pg pro pg Protein
darauf hinweist, dass die Person/das Tier wahrscheinlich von einer Behandlung mit dieser Verbindung, oder einem pharmazeutisch annehmbaren Salz davon, profitiert.

7. Verfahren nach einem der Ansprüche 5 oder 6, wobei ein H₂O₂-Spiegel von mehr als 20.000 Relativen Fluoreszenzintensität anzeigt, dass die Person/das Tier wahrscheinlich von einer Behandlung mit einer Verbindung der Formel (I), oder einem pharmazeutisch annehmbaren Salz davon, profitiert.

8. Verbindung der Formel (I), oder ein pharmazeutisch annehmbares Salz davon, zur Verwendung nach einem der Ansprüche 1 bis 4 oder ein Verfahren nach einem der Ansprüche 5 bis 7, wobei der GSH-Spiegel durch Lumineszenz bestimmt ist.

9. Verbindung der Formel (I), oder ein pharmazeutisch annehmbares Salz davon, zur Verwendung nach Anspruch 4 oder ein Verfahren nach einem der Ansprüche 6 bis 8, wobei der Spiegel an MDA-Addukten und/oder HNE-Addukten durch Immunmonitoring bestimmt ist.

10. Verbindung der Formel (I), oder ein pharmazeutisch annehmbares Salz davon, zur Verwendung nach einem der Ansprüche 1 bis 4 und 8 bis 9 oder ein Verfahren nach einem der Ansprüche 5 bis 9, wobei der Krebs ausgewählt ist aus Blasenkrebs, Hirntumoren, Brustkrebs, Melanom, multiplem Myelom, Leukämie, Lymphom, Prostatakrebs, Gebärmutterhalskrebs, Magenkrebs, Leberkrebs, Zungenkrebs, Eierstockkrebs, Bauchspeicheldrüsenkrebs, Nierenkrebs, Pleuramesotheliom, Osteosarkom, Muskelkrebs, Lungenkrebs, Nierenkrebs, Kopf- und Halskrebs, Darmkrebs, Blutkrebs, Krebs des zentralen Nervensystems und Sarkom.

11. Verbindung zur Verwendung nach einem der Ansprüche 1 bis 4 und 8 bis 10 oder ein Verfahren nach einem der Ansprüche 5 bis 10, **dadurch gekennzeichnet, dass** in der Verbindung der Formel (I) X1 und X2, identisch oder unterschiedlich, ausgewählt sind aus einem Methyl, einem Phenyl, einem Benzyl, wobei mindestens eines aus X1 oder X2 ein Methyl ist, oder X1 und X2 zusammen mit dem Stickstoffatom, an das sie gebunden sind, ein Piperidin oder ein Morpholin bilden.

12. Verbindung zur Verwendung nach einem der Ansprüche 1 bis 4 und 8 bis 11 oder ein Verfahren nach einem der Ansprüche 5 bis 11, wobei die Verbindung der Formel (I) ausgewählt ist aus:
- S-Methyl-4-methyl-4-(piperidin-1-yl)pent-2-ynethioat;
- S-Methyl-4-[benzyl(methyl)amino]-4-methylpent-2-ynethioat;
- S-Methyl-4-methyl-4-[methyl(phenyl)amino]pent-2-ynethioat;
- S-Methyl-4-methyl-4-(morpholin-4-yl)pent-2-ynethioat; und
- S-Methyl-4-(dimethylamino)-4-methylpent-2-ynethioat.

13. Verbindung zur Verwendung nach einem der Ansprüche 1 bis 4 und 8 bis 12 oder ein Verfahren nach einem der Ansprüche 5 bis 12, wobei die Verbindung der Formel (I) das S-Methyl-4-(dimethylamino)-4-methylpent-2-ynethioat, oder ein pharmazeutisch annehmbares Salz davon, ist.

14. Verbindung zur Verwendung nach Anspruch 13 oder ein Verfahren nach Anspruch 13, wobei das pharmazeutisch annehmbare Salz 4-(Dimethylamino)-4-methyl-2-pentynethiosäure-S-methylesterfumarat ist.

## Revendications

1. Composé de formule (I) : 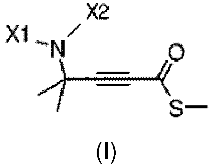 dans laquelle X1 et X2, identiques ou différents, sont choisis parmi un groupe alkyle en C₁ à C₇, un phényle, un benzyle, ou bien X1 et X2, conjointement avec l'atome d'azote auquel ils sont liés, forment un hétérocycle, en particulier une pipéridine ou une morpholine ;
ou un sel pharmaceutiquement acceptable de celui-ci ;
pour son utilisation pour le traitement d'un cancer chez un sujet ;
dans lequel les cellules cancéreuses dudit sujet :
- ont un niveau accru de H₂O₂ d'au moins 200 % en comparaison avec le niveau de référence de H₂O₂ dans des cellules normales provenant du même tissu que les cellules cancéreuses ; et
- ont un niveau de GSH inférieur à 0,5 nmol pour 25 000 cellules,
et dans lequel ledit sujet est identifié par mesure du niveau de H₂O₂ et du niveau de GSH dans les cellules cancéreuses dudit sujet.

2. Composé de formule (I) ou un sel pharmaceutiquement acceptable de celui-ci pour son utilisation selon la revendication 1, dans lequel ledit niveau de H₂O₂ est déterminé par quantification du niveau d'intensité de fluorescence.

3. Composé de formule (I) ou un sel pharmaceutiquement acceptable de celui-ci pour son utilisation selon la revendication 2, dans lequel ledit niveau de H₂O₂ est supérieur à 20 000 intensités de fluorescence relative.

4. Composé de formule (I) ou un sel pharmaceutiquement acceptable de celui-ci pour son utilisation selon l'une quelconque des revendications 1 et 2, dans lequel lesdites cellules cancéreuses dudit sujet ont aussi un niveau d'adduits de MDA supérieur à 75 ng par µg de protéine totale et/ou un niveau d'adduits de HNE supérieur à 1 µg par µg de protéine totale, après traitement *in vitro* avec un composé de formule (I) ou un sel pharmaceutiquement acceptable de celui-ci.

5. Procédé pour sélectionner un sujet souffrant d'un cancer et qui va le plus vraisemblablement tirer bénéfice d'un traitement avec un composé de formule (I) : 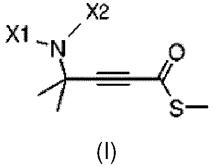 dans laquelle X1 et X2, identiques ou différents, sont choisis parmi un groupe alkyle en C₁ à C₇, un phényle, un benzyle, ou bien X1 et X2, conjointement avec l'atome d'azote auquel ils sont liés, forment un hétérocycle, en particulier une pipéridine ou une morpholine ;
ou un sel pharmaceutiquement acceptable de celui-ci ;
lequel procédé comprend :
a. la mesure du niveau de H₂O₂ dans un échantillon de cellules cancéreuses dudit sujet ;
b. la comparaison du niveau résultant de l'étape a. avec le niveau de référence de H₂O₂ dans des cellules normales provenant du même tissu que les cellules cancéreuses ; et
la mesure du niveau de GSH dans un échantillon de cellules cancéreuses dudit sujet ;
dans lequel :
- un niveau accru de H₂O₂ d'au moins 200 % en comparaison avec le niveau de référence de H₂O₂ dans des cellules normales provenant du même tissu que les cellules cancéreuses ; et
- un niveau de GSH dudit échantillon de cellules cancéreuses du sujet inférieur à 0,5 nmol pour 25 000 cellules,
indiquent que le sujet est susceptible de tirer bénéfice d'un traitement avec ledit composé ou un sel pharmaceutiquement acceptable de celui-ci.

6. Procédé selon la revendication 5, lequel procédé comprend :
a) la mesure du niveau de H₂O₂ dans un échantillon de cellules cancéreuses dudit sujet ;
b) la comparaison du niveau résultant de l'étape a. avec le niveau de référence de H₂O₂ dans des cellules normales provenant du même tissu que les cellules cancéreuses ;
c) la mesure du niveau de GSH dans un échantillon de cellules cancéreuses dudit sujet ; et
d) la mesure du niveau d'adduits de MDA et/ou du niveau d'adduits de HNE après un traitement *in vitro* avec un composé de formule (I) ou un sel pharmaceutiquement acceptable de celui-ci dans un échantillon de cellules cancéreuses dudit sujet ;
dans lequel :
- un niveau accru de H₂O₂ d'au moins 200 % en comparaison avec le niveau de référence de H₂O₂ dans des cellules normales provenant du même tissu que les cellules cancéreuses,
- un niveau de GSH dudit échantillon de cellules cancéreuses du sujet inférieur à 0,5 nmol pour 25 000 cellules, et
- un niveau d'adduits de MDA supérieur à 75 ng par µg de protéine totale et/ou un niveau d'adduits de HNE supérieur à 1 µg par µg de protéine totale,
indiquent que le sujet est susceptible de tirer bénéfice d'un traitement avec ledit composé ou un sel pharmaceutiquement acceptable de celui-ci.

7. Procédé selon l'une quelconque des revendications 5 et 6, dans lequel un niveau de H₂O₂ supérieur à 20 000 intensités de fluorescence relative indique que le sujet est susceptible de tirer bénéfice d'un traitement avec un composé de formule (I) ou un sel pharmaceutiquement acceptable de celui-ci.

8. Composé de formule (I) ou un sel pharmaceutiquement acceptable de celui-ci pour son utilisation selon l'une quelconque des revendications 1 à 4 ou procédé selon l'une quelconque des revendications 5 à 7, dans lequel le niveau de GSH est déterminé par luminescence.

9. Composé de formule (I) ou un sel pharmaceutiquement acceptable de celui-ci pour son utilisation selon la revendication 4 ou procédé selon l'une quelconque des revendications 6 à 8, dans lequel le niveau d'adduits de MDA et/ou le niveau d'adduits de HNE sont déterminés par immuno-surveillance.

10. Composé de formule (I) ou un sel pharmaceutiquement acceptable de celui-ci pour son utilisation selon l'une quelconque des revendications 1 à 4 et 8 à 9 ou procédé selon l'une quelconque des revendications 5 à 9, dans lequel le cancer est choisi parmi un cancer de la vessie, une tumeur au cerveau, un cancer du sein, un mélanome, un myélome multiple, une leucémie, un lymphome, un cancer de la prostate, un cancer du col, un cancer de l'estomac, un cancer du foie, un cancer de la langue, un cancer ovarien, un cancer du pancréas, un cancer rénal, une pleuramésothélomie, un ostéosarcome, un cancer musculaire, un cancer du poumon, un cancer du rein, un cancer de la tête et du cou, un cancer du côlon, un cancer du sang, un cancer du système nerveux central, et un sarcome.

11. Composé pour son utilisation selon l'une quelconque des revendications 1 à 4 et 8 à 10 ou procédé selon l'une quelconque des revendications 5 à 10, dans lequel, dans ledit composé de formule (I), X1 et X2, identiques ou différents, sont choisis parmi un méthyle, un phényle, un benzyle, au moins l'un de X1 et X2 étant un méthyle, ou bien X1 et X2, conjointement avec l'atome d'azote auquel ils sont liés, forment une pipéridine ou une morpholine.

12. Composé pour son utilisation selon l'une quelconque des revendications 1 à 4 et 8 à 11 ou procédé selon l'une quelconque des revendications 5 à 11, dans lequel le composé de formule (I) est choisi parmi :
- le 4-méthyl-4-(pipéridin-1-yl)pent-2-ynethioate de S-méthyle ;
- le 4-[benzyl(méthyl)amino]-4-méthylpent-2-ynethioate de S-méthyle ;
- le 4-méthyl-4-[méthyl(phényl)amino]pent-2-ynethioate de S-méthyle ;
- le 4-méthyl-4-(morpholin-4-yl)pent-2-ynethioate de S-méthyle ; et
- le 4-(diméthylamino)-4-méthylpent-2-ynethioate de S-méthyle.

13. Composé pour son utilisation selon l'une quelconque des revendications 1 à 4 et 8 à 12 ou procédé selon l'une quelconque des revendications 5 à 12, dans lequel ledit composé de formule (I) est le 4-(diméthylamino)-4-méthylpent-2-ynethioate de S-méthyle ou un sel pharmaceutiquement acceptable de celui-ci.

14. Composé pour une utilisation selon la revendication 13 ou procédé selon la revendication 13, dans lequel ledit sel pharmaceutiquement acceptable est le fumarate d'ester S-méthylique d'acide 4-(diméthylamino)-4-méthylpent-2-pentynethioïque.
